# EUROPEAN PATENT SPECIFICATION

(11) **EP 3 743 484 B1**
(45) Date of publication and mention of the grant of the patent: **18.11.2026**
(21) Application number: 19700940.0
(22) Date of filing: 21.01.2019
(51) Int. Cl.: C09K 19/04, C09K 19/20, C09K 19/34, C09K 19/32

(54) **DIELECTRIC MATERIALS**
DIELEKTRISCHE MATERIALIEN
MATÉRIAUX DIÉLECTRIQUES

(30) Priority: 22.01.2018 EP 18152777
(43) Date of publication of application: 02.12.2020
(73) Proprietor: Merck Patent GmbH, 64293 Darmstadt (DE)
(72) Inventor: MEYER, Frank, 61479 GLASHUETTEN (DE); LARBIG, Gregor, 63571 GELNHAUSEN (DE); KOPPE, Karsten, 64293 DARMSTADT (DE)
(74) Representative: Merck Patent Association
(86) International application number: PCT/EP2019/051317
(87) International publication number: WO 2019/141833

(56) References cited:
- WO-A1-2009/158488
- WO-A1-2014/090369
- WO-A1-2017/148567
- US-A1- 2008 075 961
- US-A1- 2008 210 375
- US-A1- 2010 014 010
- US-A1- 2011 049 731
- US-B2- 8 687 259

## Description

### Technical field of the invention

The present invention relates to a novel class of polymers which can be used as dielectric material for the preparation of passivation layers in packaged electronic devices. The polymers are prepared from polymerizable compounds which have mesogenic groups. Such compounds are also referred to as reactive mesogens (RMs). The polymers obtained therefrom are thus materials with liquid-crystalline features conserved in their structure and they provide excellent film forming capability and excellent mechanical properties such as e.g. good elongation at break and good tensile strength properties. In addition, the polymers are characterized by a low dielectric constant and a low coefficient of thermal expansion (CTE). Beyond that, they are photostructurable and particularly suitable for various applications in electronic packaging such as e.g. the passivation of conductive or semiconducting components and die attach and as a major component in the preparation of substrates for printed circuit boards.

The polymerizable compounds, from which the polymers are formed, have an oligomeric structure with a repeating unit containing a mesogenic group (MG) in the middle of the molecule and polymerizable groups (P), one at each end of the molecule. There is further provided a method for forming said polymers and an electronic device containing said polymers as dielectric material. Beyond that, the present invention relates to a manufacturing method for preparing a packaged microelectronic structure, wherein a dielectric polymer layer is formed from said polymerizable compounds, and to a microelectronic device comprising said packaged microelectronic structure which is obtained or obtainable by said manufacturing method.

The manufacturing method according to the present invention allows a cost-effective and reliable manufacturing of microelectronic devices where the number of defective products caused by mechanical deformation (warping) due to undesirable thermal expansion is significantly reduced. Polymerization can occur at significant lower temperatures and thus leading to lower thermal stress during manufacturing, which reduces the waste of defective microelectronic devices which allows a resource-efficient and sustainable production.

### Background of the invention

Reactive mesogens (RMs), when polymerized at temperatures at which they exhibit thermotropic liquid crystal (LC) phases (typically nematic, cholesteric or smectic), give anisotropic polymers which conserve the liquid crystalline state. In particular, optical anisotropy has been widely exploited in the field of optical films for compensation and brightness enhancement in flat panel displays, especially in liquid crystal displays.

In addition to their wide use in liquid crystal displays and displays, liquid crystalline materials are investigated for their advantages in other application types due to their special physical properties (R. Stannarius, Nat. Mat. 2009, 8, 617-618; and J.P.F. Lagerwall et al., Current Appl. Phys. 2012, 12, 1387-1412). In particular, highly ordered anisotropic polymer networks are an interesting material class with a diverse range of applications (D.J. Broer et al., Lagmuir 2014, 30, 13499-13509; and R. Zentel et al., Adv. Mater. 2010, 22, 3366-3387). However, in most cases the LC polymers or corresponding monomers from which the polymers are made do not have optimum properties required for the respective application.

WO 2012/152409 A1 relates to polymer particles with optical anisotropy and shape anisotropy comprising monomer units of at least one reactive mesogen, a process for their preparation, the use of these particles for the preparation of optical, electrooptical, electronic, electrochemical, electrophotographic, electrowetting and electrophoretic displays and/or devices and security, cosmetic, decorative, and diagnostic applications, and electrophoretic fluids and displays comprising said polymer particles. In particular, the polymer particles have monomer units of at least one RM with at least two polymerizable groups, at least one polymerizable dye as co-monomer, optionally at least one co-monomer, optionally at least one cross-linking co-monomer, optionally at least one ionic co-monomer, and optionally at least one polymerizable stabilizer.

Various processes for the preparation of liquid-crystalline polymers from RMs and for the preparation of the RM starting materials are known from the state of the art. For example, Siemensmeyer et al. describes a process for producing mixtures of LC compounds, wherein at least one of the starting components consists of a mixture of at least two compounds and this mixture is reacted with at least one other starting component to form a statistic mixture (WO 96/04351 A1).

In addition to efficient preparation methods for LC compounds, suitable polymerization methods for forming anisotropic polymer networks are in the focus of interest. A variety of reactive functional groups were investigated for their applicability in photoinitiated polymerization reactions. The most widespread reactive functional groups are acrylates and methacrylates, which are particularly well suited for UV-induced free-radical polymerizations due to their high polymerization rate (D.J. Broer et al., Lagmuir 2014, 30, 13499-13509). However, UV curing is not suitable for all kinds of application.

Mixtures of polymerizable liquid-crystalline monomers (reactive mesogens) can be used to prepare thin films which can be cured by thermal or photoinitiated polymerization. Films which are prepared in this way are relatively thin and contain a highly cross-linked duroplastic polymer which has a pronounced dimensional stability. However, these films are relatively brittle and exhibit very low elasticity. If, on the other hand, the crosslinking degree is reduced, no dimensionally stable polymer films can be obtained.

US 6,261,481 B1 describes an organic insulating composition which provides a good thermal conductivity. The insulating composition contains a liquid crystal (LC) resin comprising a polymerization product of a resin composition containing a monomer which has a mesogenic group. The composition has a thermal conductivity in directions mutually orthogonal to each other of ≥ 0.4 W/mK. The monomer contained in the resin composition has a mesogenic group and preferably an epoxy group which can be polymerized thermally under acid catalysis. Preferably, the resin composition is heated under conditions that the monomer having the mesogenic group is partially arranged when the polymerization starts so that the anisotropic properties based on the partial arrangement are frozen in the polymer.

US 2008/0075961 A1 and US 2017/0152418 A1 relate to maleimide adhesive films which are prepared from thermosetting maleimide resins containing imide-extended mono-, bis- and polymaleimide compounds. The maleimide adhesive films are photostructurable and suitable for the production of electronic equipments, integrated circuits, semiconductor devices, passive devices, solar batteries, solar modules, and/or light emitting diodes. However, the maleimide compounds do not contain any mesogenic groups which could impart a preferential direction or partial arrangement of the compound in the film. This results in poorer properties with regard to mechanical stability and thermal conductivity. These materials typically also exhibit a relatively low glass transition point, which again impact their thermal expansion properties.

KR 20160052234 A describes a photocurable insulating resin composition and printed circuit board using the same. The photocurable insulating resin composition comprises a photocurable liquid crystal oligomer; a photocurable graphene oxide; and a photocurable metal alkoxide. However, the photocurable liquid crystal oligomers do not contain multiple mesogenic groups linked together by a spacer group. This results in an unfavorable solubility profile and energies used for photocuring are very high.

Further polymerizable compounds are known from WO 2017/148567 A1, WO 2014/090369 A1, US 2008/210375 A1, US 8 687 259 B2 and WO 2009/158488 A1, for instance.

### Electronic packaging

As solid-state transistors started to replace vacuum-tube technology, it became possible for electronic components, such as resistors, capacitors, and diodes, to be mounted directly by their leads into printed circuit boards of cards, thus establishing a fundamental building block or level of packaging that is still in use. Complex electronic functions often require more individual components than can be interconnected on a single printed circuit card. Multilayer card capability was accompanied by development of three-dimensional packaging of daughter cards onto multilayer mother boards. Integrated circuitry allows many of the discrete circuit elements such as resistors and diodes to be embedded into individual, relatively small components known as integrated circuit chips or dies. In spite of incredible circuit integration, however, more than one packaging level is typically required, in part because of the technology of integrated circuits itself. Integrated circuit chips are quite fragile, with extremely small terminals. First-level packaging achieves the major functions of mechanically protecting, cooling, and providing capability for electrical connections to the delicate integrated circuit. At least one additional packaging level, such as a printed circuit card, is utilized, as some components (high-power resistors, mechanical switches, capacitors) are not readily integrated onto a chip. For very complex applications, such as mainframe computers, a hierarchy of multiple packaging levels is required.

A wide variety of advanced packaging technologies exist to meet the requirements of today's semiconductor industry. The leading Advanced Packaging technologies - wafer-level packaging (WLP), fan-out wafer level packaging (FOWLP), 2.5D interposers, chip-on-chip stacking, package-on-package stacking, embedded IC - all require structuring of thin substrates, redistribution layers and other components like high resolution interconnects. The end consumer market presents constant push for lower prices and higher functionality on ever smaller and thinner devices. This drives the need for the next generation packaging with finer features and improved reliability at a competitive manufacturing cost.

Wafer-level packaging (WLP) is the technology of packaging an integrated circuit while still part of the wafer, in contrast to the more conventional method of slicing the wafer into individual circuits (dice) and then packaging them. WLP is essentially a true chip-scale package (CSP) technology, since the resulting package is practically of the same size as the die. Wafer-level packaging allows integration of wafer fab, packaging, test, and burn-in at wafer level in order to streamline the manufacturing process undergone by a device from silicon start to customer shipment. Major application areas of WLP are smartphones and wearables due to their size constraints. Functions provided WLPs in smartphones or wearables include: compass, sensors, power management, wireless etc. Wafer-level chip scale packaging (WL-CSP) is one of the smallest packages currently available on the market.

Fan-out wafer level packaging (FOWLP) is one of the latest packaging trends in microelectronics: FOWLP has a high miniaturization potential both in the package volume as well as in the packaging thickness. Technological basis of FOWLP is a reconfigured, painted wafer with embedded chips and a thin film rewiring layer, which together form a surface-mounted device (SMD)-compatible package. The main advantages of the FOWLP are a very thin, because substrateless package, the low thermal resistance, good high-frequency properties due to short and planar electrical connections together with a bumpless chip connection instead of e.g. wire bonds or solder contacts.

For electronic packaging, different types of material are needed, from which the substrate, electrically conductive connections and insulating or passivating coatings are made. Substrate insulator materials for multichip and single-chip modules are selected from one of two broad groups of materials, organics and ceramics. Chip-to-package interconnection is typically achieved by one of three techniques: wire bond, tape-automated bond, and solder-ball flip chip. The wire bond is the most widely used first-level interconnection; it employs ultrasonic energy to weld very fine wires mechanically from metallized terminal pads along the periphery of the integrated circuit chip to corresponding bonding pads on the surface of the substrate.

Another important role play polymers which are key building blocks for all WLP and related technologies such as IPD (integrated passives devices) and 3D-SiP (system in Package). A couple of different classes of photo-sensitive polymeric materials are available for the integration, for example: polyimide (PI), polybenzoxazole (PBO), benzocyclobutene (BCB), silicones, acrylates and epoxy resins.

Polymeric coatings such as polyurethanes, acrylic, epoxy and silicone resins have been used for over 40 years to protect printed wiring boards (PWB) from moisture, handling and environmental influences. Special semiconductor grade polymers have been developed for chip passivation layers. Especially for epoxy resins multiple distillation procedures were introduced to remove the sodium and chloride ions which are by-products in the standard epoxy synthesis. Polyimide became the standard passivation layer for memory chips and other devices with the need of surface protection for the handling and testing procedure. Photo-sensitive resins have been developed to reduce processing cost.

Dry-etching requires a masking process with either a hard mask which is a sputtered and structured metal layer or a thick photo resist coating. Due to cost saving programs the in-depth characterization of material that was prevalent in the 1960s through the 1990s has slowed down considerably. In addition, due to mergers, spin-offs and low economic margins, the continuity of polymeric products is not always given. In contrast to that, polymeric materials became more important with the introduction of new packaging concepts for ICs like WLP, SiP and 3-D integration.

There is a wide range of material classes which could be used as a polymeric dielectric for WLP: polyimide, polybenzoxazole, benzocyclobutene, epoxy, silicone, acrylate, nanofilled phenol resin, siloxane, fluorinated polymer and polynorbornene. A profound overview of photo-sensitive formulations is given in M. Töpper et al., 2010 Electronic Components and Technology Conference, 769-776.

Polyimide-ODA was the first member of a series of new high performance polymers based on alternate aromatic homocyclic and heterocyclic rings developed by Du Pont:

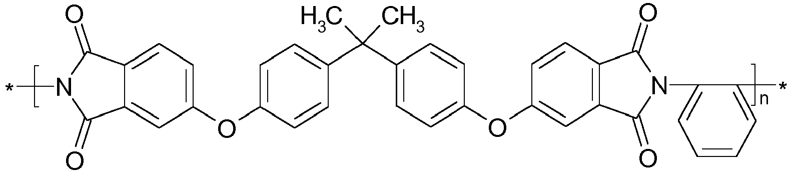

Polyimides are quite unique to their very high decomposition temperature which can go up to over 400°C. In addition, their mechanical properties guarantee a high flexibility (elongation at break up to 100%) with a very high tensile strength of over 200 MPa. PI is still the most popular polymer for IC passivation.

A modification to the negative-sensitive polymer PI was achieved by polybenzoxazole (PBO) which is sometime also called a positive-sensitive PI:

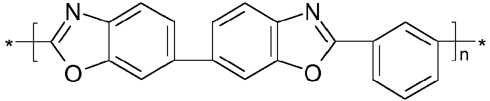

The polymer film can be developed after photo-exposure using an aqueous developer.

Siloxanes are polymers which have a back-bone of Si-O-groups. The so-called BCB (benzocyclobutene) is an example of a siloxane-polymer group having in addition a vinyl and a benzocyclobutene ring system:

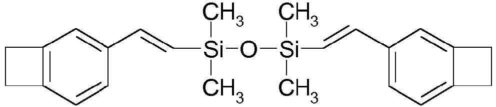

A major advantage is the polymerization reaction (Diels-Alder reaction) which has high atomic economy, since no by-products occur. This highly-crosslinked thermoset polymer has excellent electrical performance, but is quite brittle with a low elongation to break value (8%) and low tensile strength of 87 MPa.

Epoxy polymers are the basis for most PWBs. A major advantage is their low curing temperature suitable for temperature sensitive devices. Important examples are Novolak-based epoxy resins:

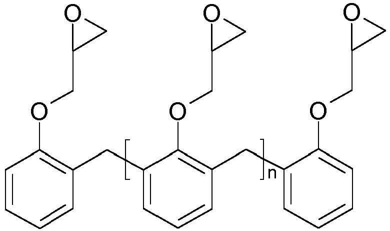

Epoxy and acrylate polymers are also available as dry-film materials. All other dielectrics have to be deposited by spin or spray coating.

Newer developments are polynorbornenes which are bridged cyclic hydrocarbons giving polymers by ring-opening metathesis polymerization or vinyl-addition polymerization:

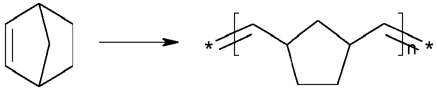

The processing of all liquid type photo-sensitive thin film polymers is quite similar: coating (mostly spin coating), exposure (UV), post exposure bake (not all polymers), development, post exposure bake and cure.

The requirements for the selection of a given polymer are quite broad: high decomposition or glass temperature for high temperature processes in packaging like solder reflow, high adhesion, high mechanical and chemical strength, excellent electrical properties, low water uptake, photosensitivity and high yield manufacturability. For these reasons, thermosets are usually used for packaging applications. They are manufactured and employed as pre-polymers which have a molecular weight of several 100.000 g/mol and are dissolved in an organic solvent. Such solutions are commonly called pre-cursors and are ready for the spin coating process. The final polymerization is performed on the wafer by thermal curing.

However, the polymers known from the state of the art show the following drawbacks:
- Polyimides and polybenzoxazoles typically require very high processing temperatures which increase the risk for warping, especially in multilayer redistribution layers (RDLs). In addition to that, polyimides show a high water uptake which is a problem for reliability of the devices.
- Benzocyclobutene derivatives as well as polynorbornenes show a very low dielectric constant, but this advantage is compromised by very poor adhesion to metals.

### Photolithography

Photolithography has long been the key patterning technology for structuring inorganic and organic materials used in advanced packaging applications like flip-chip wafer bumping, electroplated gold, solder bumps, copper pillar technologies and redistribution layers. Photolithography is a key manufacturing process and cost contributor; the careful selection of the right exposure solution is critical to achieve the best possible cost structure in today's industrial lithography applications.

Current drivers and trends in the semiconductor industry clearly show that performance improvements of microelectronic devices are needed to meet the future end user requirements. For example, consumer electronic devices like tablets and smartphones are getting thinner and smaller while gaining higher computing power, increased data storage, and improved communication capabilities. In addition, cost considerations become more and more important in the competitive landscape for all parties within the supply chain, from the chip manufacturers, foundries, assembly and test suppliers to the device manufacturers. Therefore, the industry strives for innovative approaches to lower manufacturing costs coupled with enabling technologies that meet the challenging technical requirements.

For decades, photolithography has been and still is the fundamental process used in the fabrication and packaging of microelectronic devices. A key component of any photolithography process is the exposure tool, which uses light in the ultraviolet wavelength range to pattern a photosensitive resist or polymer. The exposure tool must be able to precisely create the desired features and align them to the previously fabricated structures in the underlying layers. Several types of exposure technologies exist today: proximity or contact printing, laser direct imaging, and projection lithography. The corresponding equipment toolsets differ in terms of technical capability (optical resolution, overlay performance and effective throughput), and the costs related to the exposure process. (see H. Hichri et al., SÜSS Micro Tec Photonic System Inc., Corona, CA, USA).

### Object of the invention

Hence, it is an object of the present invention to overcome the disadvantages in the prior art and to provide a new class of polymers which can be used as a dielectric material in passivation layers of packaged electronic devices showing excellent film forming capability, easy processing from conventional solvents and excellent mechanical properties such as e.g. good elongation at break and good tensile strength properties and having a low dielectric constant and a low coefficient of thermal expansion (CTE).

Moreover, it is an object of the present invention to provide a novel polymer which is photostructurable and particularly suitable for various applications in electronic packaging such as e.g. the passivation of conductive or semiconducting components and die attach and as a major component in the preparation of substrates for printed circuit boards.

It is a further object of the present invention to provide a polymerizable compound as a monomer from which the novel polymer is made of. Beyond that, it is an object of the present invention to provide a method for forming said novel polymer using the polymerizable compound. Finally, it is an object of the present invention to provide an electronic device comprising said polymer as dielectric material, a manufacturing method for preparing a packaged microelectronic structure and a microelectronic device comprising said packaged microelectronic structure which is obtainable by said manufacturing method.

### Summary of the invention

The present inventors have surprisingly found that the above objects are achieved by a polymer containing repeating units derived from a specific polymerizable compound which preferably is a polymerizable direactive mesogenic compound.

The polymerizable compound is represented by Formula (1):

P¹-Sp¹-(MG-Sp¹)ₘ-P¹ Formula (1)

wherein:
- m: is an integer from 1 to 60, preferably 1 to 50, more preferably 2 to 30, and most preferably 3 to 20;
- P¹: denotes a polymerizable group (P);
- Sp¹: denotes at each occurrence a spacer group (Sp) or a single bond;
- MG: is a rod-shaped mesogenic group, which is preferably selected from Formula (2):

-(A²¹-Z²¹)ₖ-A²²-(Z²²-A²³)ₗ- Formula (2)

wherein:
- A²¹ to A²³: are independently and at each occurrence independently of one another an aryl group, heteroaryl group, heterocyclic group, alicyclic group or cyclic imide group optionally being substituted by one or more identical or different groups L;
- Z²¹ and Z²²: are independently and at each occurrence independently from each other, -O-, -S-, -CO-, -COO-, -OCO-, -S-CO-, -CO-S-, -O-COO-, -CO-NR⁰¹-, -NR⁰¹-CO-, -NR⁰¹-CO-NR⁰², -NR⁰¹-COO-, -O-CO-NR⁰¹-, -OCH₂-, -CH₂O-, -SCH₂-, -CH₂S-, -CF₂O-, -OCF₂-, -CF₂S-, -SCF₂-, -CH₂CH₂-, -(CH₂)₄-, -CF₂CH₂-, -CH₂CF₂-, -CF₂CF₂-, -CH=N-, -N=CH-, -N=N-, -CH=CR⁰¹-, -CY⁰¹=CY⁰²-, -C≡-C-, -CH=CH-COO-, -OCO-CH=CH-, or a single bond;
- R⁰¹ and R⁰²: each, independently of one another, denote H or alkyl having 1 to 12 C atoms;
- L: is F, Cl, Br, I, -CN, -NO₂, -NCO, -NCS, -OCN, -SCN, -C(=O)NR^{xx}R^{yy}, -C(=O)OR^{xx}, -C(=O)R^{xx}, -NR^{xx}R^{yy}, -OH, -SF₅, or straight chain or branched chain alkyl, alkoxy, alkylcarbonyl, alkoxycarbonyl, alkylcarbonyloxy or alkoxycarbonyloxy with 1 to 20 C atoms, preferably 1 to 12 C atoms, wherein one or more H atoms are optionally replaced by F or Cl, preferably F, -CN or straight chain or branched chain alkyl, alkoxy, alkylcarbonyl, alkoxycarbonyl, alkylcarbonyloxy or alkoxycarbonyloxy with 1 to 6 C atoms;
- R^{xx} and R^{yy}: independently of each other denote H or alkyl with 1 to 12 C atoms;
- Y⁰¹ and Y⁰²: each, independently of one another, denote H, alkyl having 1 to 12 C atoms, aryl, F, Cl, or CN; and
- k and l: are each and independently 0, 1, 2, 3 or 4, preferably 0, 1 or 2, most preferably 1.

Said polymerizable compounds are used as monomer compounds to form a new class of polymers. Said new class of polymers is prepared by the following method which also forms part of the present invention:

Method for forming a polymer, wherein the method comprises the following steps:
(i) providing a polymerizable starting material comprising one or more polymerizable compounds according to the present invention; and
(ii) polymerizing said polymerizable starting material.

Moreover, a polymer is provided which is obtainable or obtained by the above-mentioned method for forming a polymer.

Beyond that, an electronic device is provided comprising a polymer according to the present invention.

Finally, a manufacturing method for preparing a packaged microelectronic structure is provided, in which a substrate is provided with a coating comprising one or more polymerized liquid crystalline monomers, wherein the method comprises the following steps:
(1) applying a polymerizable composition comprising one or more polymerizable compounds according to the present invention to a surface of a substrate; and
(2) curing said polymerizable composition to form a dielectric layer.

There is also provided a microelectronic device comprising a packaged microelectronic structure being obtainable or obtained by the manufacturing method according to the present invention.

Preferred embodiments of the present invention are described hereinafter and in the dependent claims.

### Brief description of the figures

The present application does not contain any figures.

### Detailed description

### Definitions

The term "liquid crystal", "mesomorphic compound", or "mesogenic compound" (also shortly referred to as "mesogen") means a compound that under suitable conditions of temperature, pressure and concentration can exist as a mesophase or in particular as a LC phase. Non-amphiphilic mesogenic compounds comprise for example one or more calamitic, banana-shaped or discotic mesogenic groups.

The term "calamitic" means a rod- or board/lath-shaped compound or group. The term "banana-shaped" means a bent group in which two, usually calamitic, mesogenic groups are linked through a semi-rigid group in such a way as not to be co-linear. The term "discotic" means a disc- or sheet-shaped compound or group.

The term "mesogenic group" or its abbreviation "MG" means a group with the ability to induce liquid crystal (LC) phase behavior. Mesogenic groups, especially those of the non-amphiphilic type, are usually either calamitic or discotic. The compounds comprising mesogenic groups do not necessarily have to exhibit an LC phase themselves. It is also possible that they show LC phase behavior only in mixtures with other compounds, or when the mesogenic compounds or the mixtures thereof are polymerized. For the sake of simplicity, the term "liquid crystal" is used hereinafter for both mesogenic and LC materials.

A calamitic mesogenic compound is usually comprising a calamitic, i.e. rod- or lath-shaped, mesogenic group consisting of one or more aromatic or alicyclic groups connected to each other directly or via linkage groups, optionally comprising terminal groups attached to the short ends of the rod, and optionally comprising one or more lateral groups attached to the long sides of the rod, wherein these terminal and lateral groups are usually selected e.g. from carbyl or hydrocarbyl groups, polar groups like halogen, nitro, hydroxy, etc., or polymerizable groups.

A discotic mesogenic compound is usually comprising a discotic, i.e. relatively flat disc- or sheet-shaped mesogenic group consisting for example of one or more condensed aromatic or alicyclic groups, like for example triphenylene, and optionally comprising one or more terminal groups that are attached to the mesogenic group and are selected from the terminal and lateral groups mentioned above.

The term "reactive mesogen" or its abbreviation "RM" means a polymerizable mesogenic or liquid crystalline compound, which is preferably a monomeric or oligomeric compound. Polymerizable compounds with one polymerizable group are also referred to as "monoreactive" compounds, compounds with two polymerizable groups as "direactive" compounds, and compounds with more than two polymerizable groups as "multireactive" compounds. Compounds without a polymerizable group are also referred to as "non-reactive" compounds.

The term "spacer" or "spacer group", also referred to as "Sp" below, is known to the person skilled in the art and is described in the literature. Unless stated otherwise, the term "spacer" or "spacer group" above and below denotes a flexible organic group, which in a polymerizable mesogenic compound ("RM") connects the mesogenic group and the polymerizable group(s).

The term "polymer" includes, but is not limited to, homopolymers, copolymers, for example, block, random, and alternating copolymers, terpolymers, quaterpolymers, etc., and blends and modifications thereof. Furthermore, unless otherwise specifically limited, the term "polymer" shall include all possible configurational isomers of the material. These configurations include, but are not limited to isotactic, syndiotactic, and atactic symmetries. A polymer is a molecule of high relative molecular mass, the structure of which essentially comprises the multiple repetition of units (i.e. repeating units) derived, actually or conceptually, from molecules of low relative mass (i.e. monomers). In the context of the present invention polymers are composed of more than 60 monomers.

The term "oligomer" is a molecular complex that consists of a few monomer units, in contrast to a polymer, where the number of monomers is, in principle, unlimited. Dimers, trimers and tetramers are, for instance, oligomers composed of two, three and four monomers, respectively. In the context of the present invention oligomers may be composed of up to 60 monomers.

The term "monomer" as used herein refers to a molecule which can undergo polymerization thereby contributing constitutional units (repeating units) to the essential structure of a polymer or an oligomer.

The term "homopolymer" as used herein stands for a polymer derived from one species of (real, implicit or hypothetical) monomer.

The term "copolymer" as used herein generally means any polymer derived from more than one species of monomer, wherein the polymer contains more than one species of corresponding repeating unit. In one embodiment the copolymer is the reaction product of two or more species of monomer and thus comprises two or more species of corresponding repeating unit. It is preferred that the copolymer comprises two, three, four, five or six species of repeating unit. Copolymers that are obtained by copolymerization of three monomer species can also be referred to as terpolymers. Copolymers that are obtained by copolymerization of four monomer species can also be referred to as quaterpolymers. Copolymers may be present as block, random, and/or alternating copolymers.

The term "block copolymer" as used herein stands for a copolymer, wherein adjacent blocks are constitutionally different, i.e. adjacent blocks comprise repeating units derived from different species of monomer or from the same species of monomer but with a different composition or sequence distribution of repeating units.

Further, the term "random copolymer" as used herein refers to a polymer formed of macromolecules in which the probability of finding a given repeating unit at any given site in the chain is independent of the nature of the adjacent repeating units. Usually, in a random copolymer, the sequence distribution of repeating units follows Bernoullian statistics.

The term "alternating copolymer" as used herein stands for a copolymer consisting of macromolecules comprising two species of repeating units in alternating sequence.

"Electronic packaging" is a major discipline within the field of electronic engineering, and includes a wide variety of technologies. It refers to inserting discrete components, integrated circuits, and MSI (medium-scale integration) and LSI (large-scale integration) chips (usually attached to a lead frame by beam leads) into plates through hole on multilayer circuit boards (also called cards), where they are soldered in place. Packaging of an electronic system must consider protection from mechanical damage, cooling, radio frequency noise emission, protection from electrostatic discharge maintenance, operator convenience, and cost.

The term "microelectronic device" as used herein refers to electronic devices of very small electronic designs and components. Usually, but not always, this means micrometer-scale or smaller. These devices typically contain one or more microelectronic components which are made from semiconductor materials and interconnected in a packaged structure to form the microelectronic device. Many electronic components of normal electronic design are available in a microelectronic equivalent. These include transistors, capacitors, inductors, resistors, diodes and naturally insulators and conductors can all be found in microelectronic devices. Unique wiring techniques such as wire bonding are also often used in microelectronics because of the unusually small size of the components, leads and pads.

Points of attachment (binding sites) of structural elements presented in this patent application may be indicated by 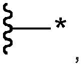 wherein * represents the structural element and 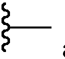 a binding site.

### Preferred embodiments

### Polymerizable compound

The present invention relates to a polymerizable compound selected from 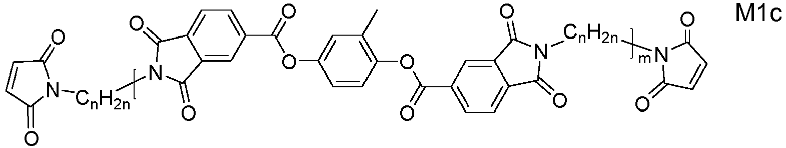 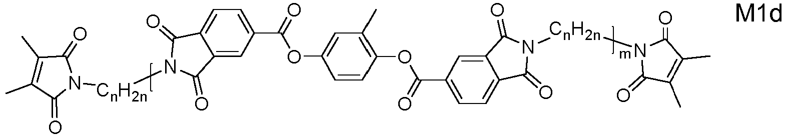 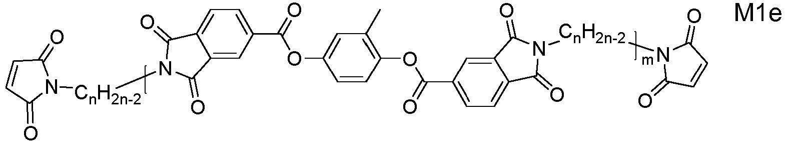 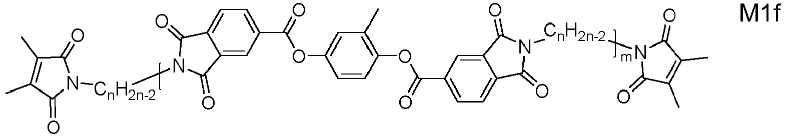 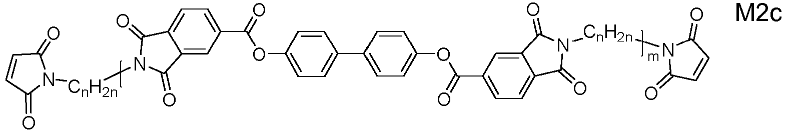 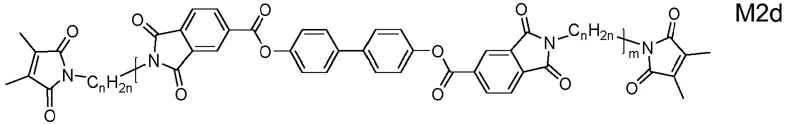 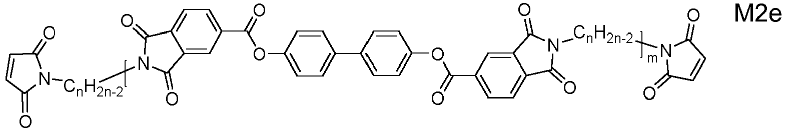 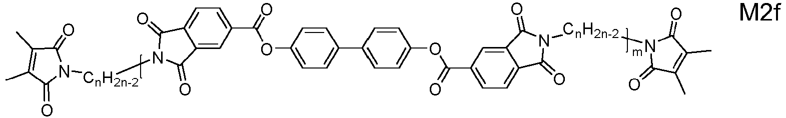 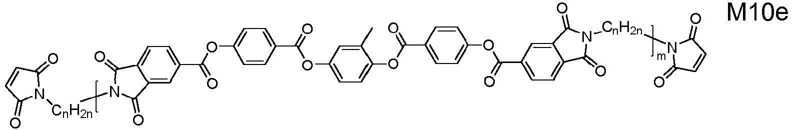 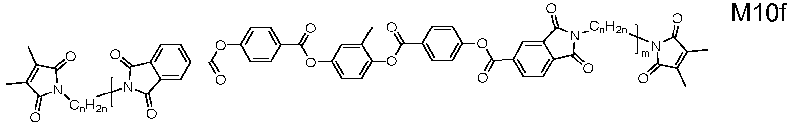 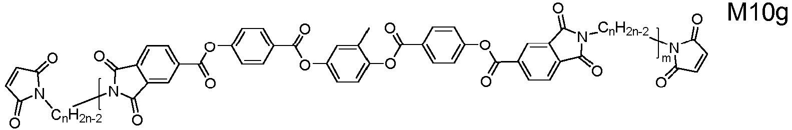 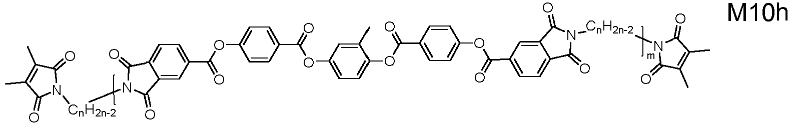 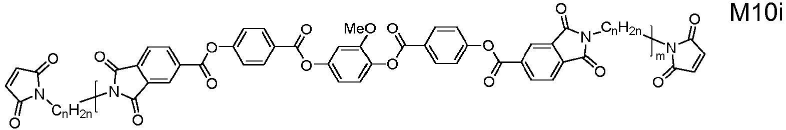 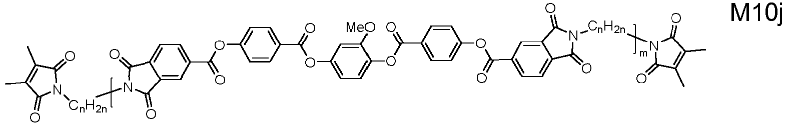 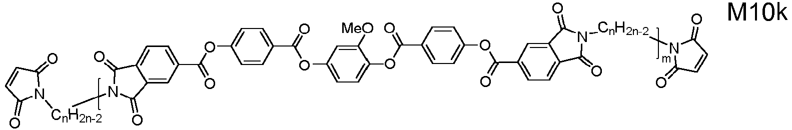 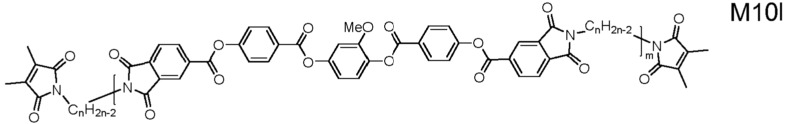 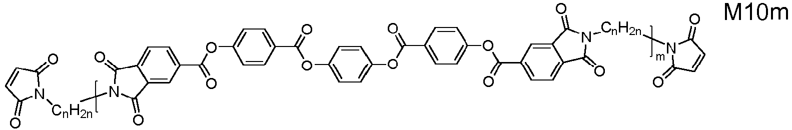 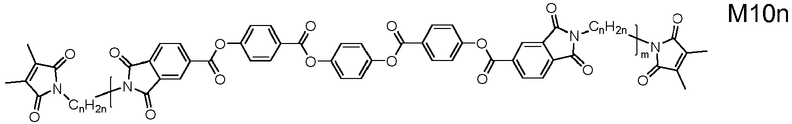 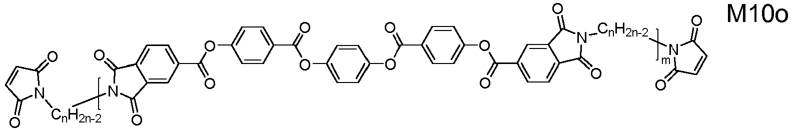 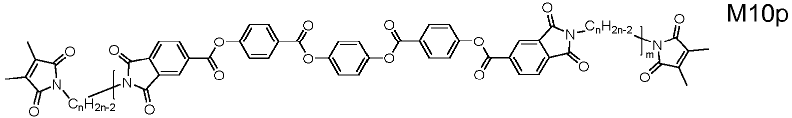 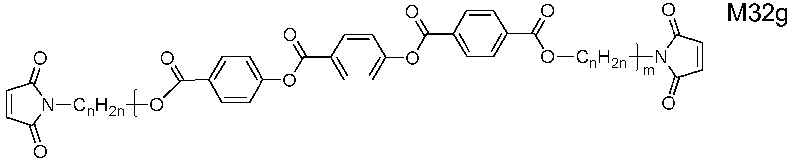 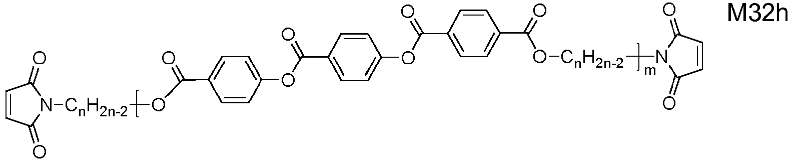 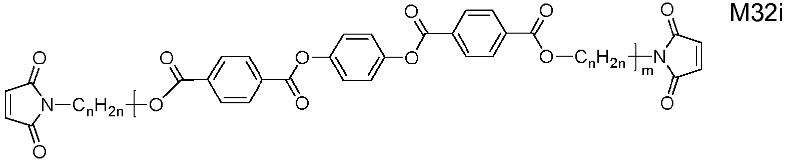 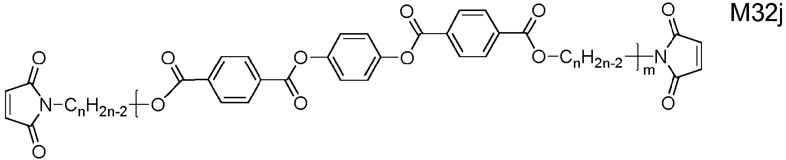 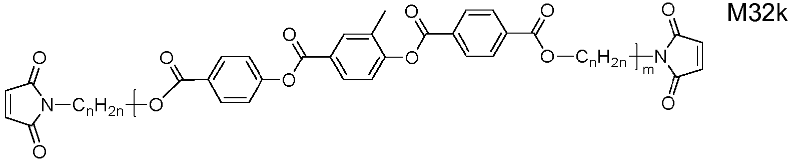 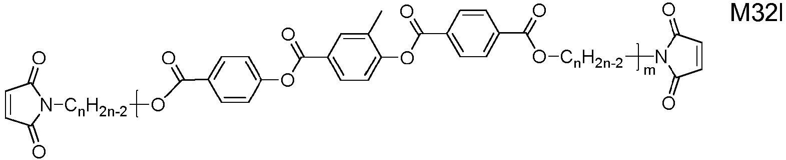 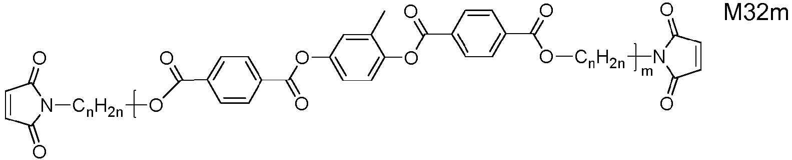 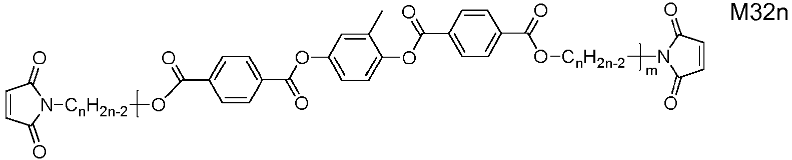 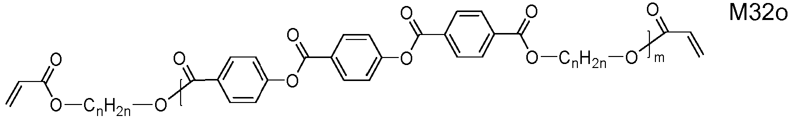 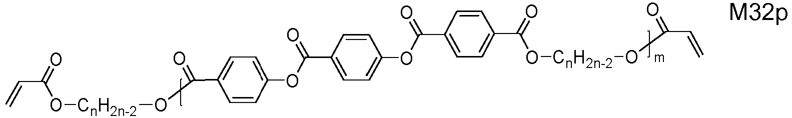 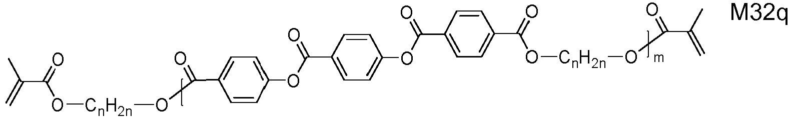 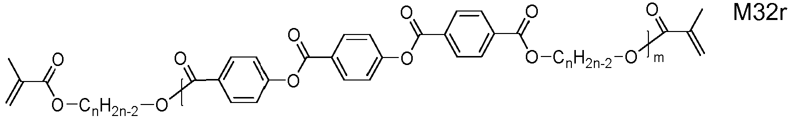 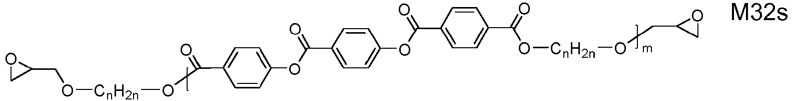 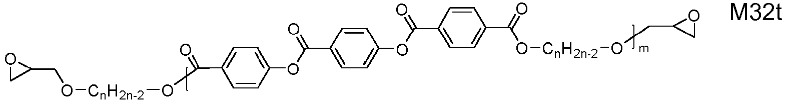 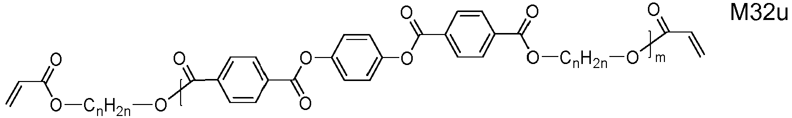 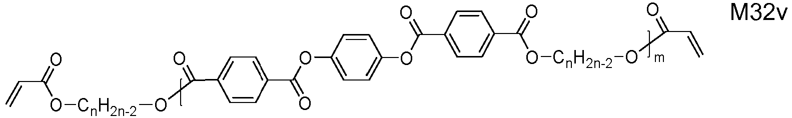 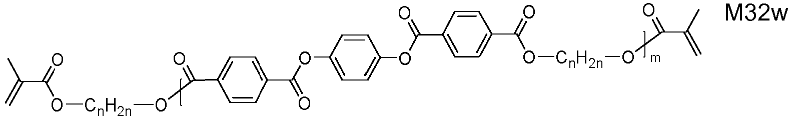 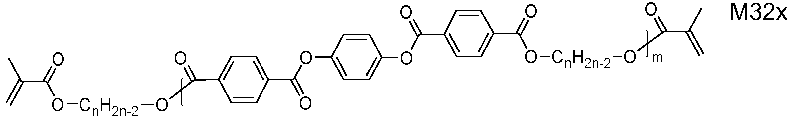 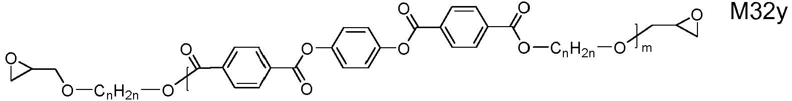 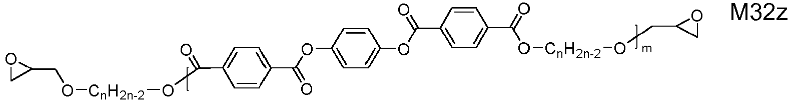 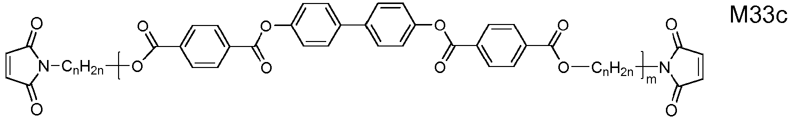 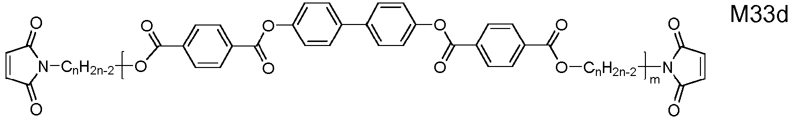 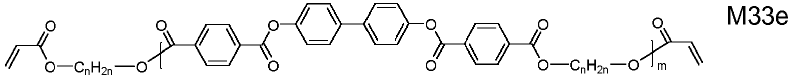 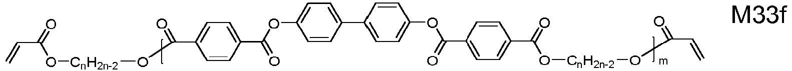 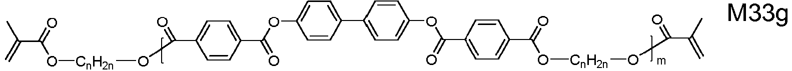 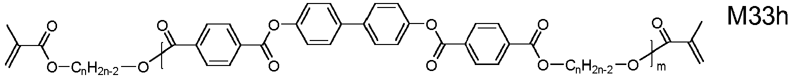 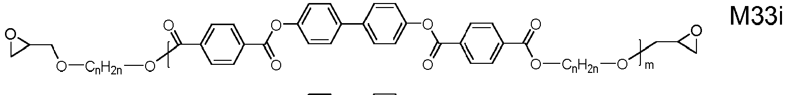 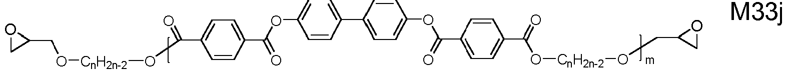 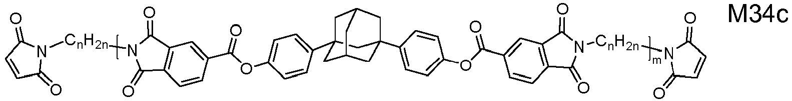 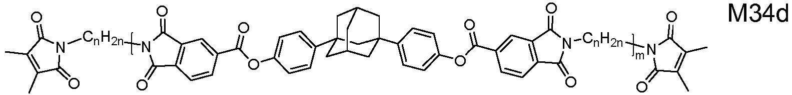 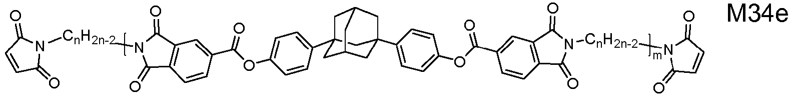 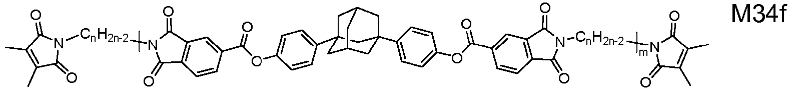 wherein
- n: is an integer from 1 to 60, preferably from 1 to 36, and more preferably from 6 to 12; and
- m: is an integer from 1 to 60, preferably 1 to 50, more preferably 2 to 30, and most preferably 3 to 20.

In the compounds of formulae M1 to M33 and the corresponding sub-formulae, the ring group 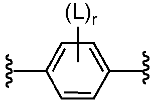 is preferably 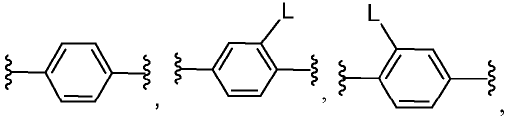 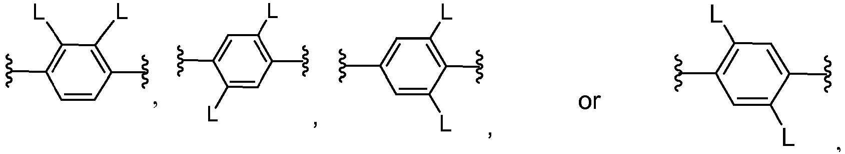 wherein
- L: is at each occurrence independently from each other F, Cl, Br, I, -CN, -NO₂, -NCO, -NCS, -OCN, -SCN, -C(=O)NR^{xx}R^{yy}, -C(=O)OR^{xx}, -C(=O)R^{xx}, -NR^{xx}R^{yy}, -OH, -SF₅, or straight chain or branched chain alkyl, alkoxy, alkylcarbonyl, alkoxycarbonyl, alkylcarbonyloxy or alkoxycarbonyloxy with 1 to 20 C atoms, preferably 1 to 12 C atoms, wherein one or more H atoms are optionally replaced by F or Cl, preferably F, -CN or straight chain or branched chain alkyl, alkoxy, alkylcarbonyl, alkoxycarbonyl, alkylcarbonyloxy or alkoxycarbonyloxy with 1 to 6 C atoms; and
- R^{xx} and R^{yy}: are defined according to the definitions given above.

The polymerizable compound of the present invention can be prepared by any standard synthesis. Usually, the compound is retrosynthetically cut into smaller units and formed stepwise from suitable precursor compounds. For this purpose, known standard reactions can be used. It has proven to be particularly advantageous to attach the polymerizable groups P¹ at a late stage of the synthesis, typically at the very last step of the synthesis. By doing so, undesirable side-reactions or premature polymerization of the compound can be avoided.

The present invention further provides a method for forming a polymer comprising monomer units of one or more of the polymerizable compound. The polymer is a dielectric polymer which may be linear or crosslinked. The method for forming a polymer comprises the following steps:
(i) providing a polymerizable starting material comprising one or more polymerizable compounds according to the present invention; and
(ii) polymerizing said polymerizable starting material.

The polymerizable compound comprised in the polymerizable starting material is a polymerizable compound of the present invention and the above-mentioned definitions.

It is preferred that the polymerizable starting material provided in step (i) comprises one polymerizable compound according to the present invention. In a preferred embodiment, the polymerizable starting material provided in step (i) comprises one or more additional polymerizable compounds which are capable to react with the polymerizable compound according to the present invention to form a copolymer.

Preferred additional polymerizable compounds are monomers which are capable to react with the polymerizable group P of the polymerizable compound of the present invention to form a copolymer. Such preferred additional polymerizable compounds are selected from acrylates, epoxides, olefins, vinyl ethers, vinyl esters, polythiols, polyamines, and polymaleimides. Using basic chemical knowledge, the skilled person is able to find and select for a given polymerizable compound of the present invention suitable additional polymerizable compounds which are capable to react with the first mentioned to form a copolymer.

Preferred acrylates are acrylic acid, methacrylic acid, methyl acrylate, methyl methacrylate, methyl cyanoacrylate, ethyl acrylate, ethyl methacrylate, ethyl cyanoacrylate, propyl acrylate, propyl methacrylate, propyl cyanoacrylate, butyl acrylate, butyl methacrylate, butyl cyanoacrylate, pentyl acrylate, pentyl methacrylate, pentyl cyanoacrylate, hexyl acrylate, hexyl methacrylate, hexyl cyanoacrylate, heptyl acrylate, heptyl methacrylate, heptyl cyanoacrylate, octyl acrylate, octyl methacrylate, octyl cyanoacrylate, ethylene glycol dimethacrylate, 2-ethylhexyl acrylate, glycidyl methacrylate, (hydroxyethyl)acrylate, (hydroxyethyl)methacrylate, methyl 2-chloroacrylate, and methyl 2-fluoroacrylate.

Preferred epoxides are ethylene oxide, propylene oxide, butylene oxide, pentylene oxide, hexylene oxide, heptylene oxide, octylene oxide, glycidamide, glycidol,styrene oxide , 3,4-epoxytetrahydrothiophene-1,1-dioxide, ethyl 2,3-epoxypropionate, methyl 2-methylglycidate, methyl glycidyl ether, ethyl glycidyl ether, diglycidyl ether, cyclopentene oxide, cyclohexene oxide, cycloheptene oxide, cyclooctene oxide, and stilbene oxide.

Preferred olefins are ethylene, propylene, butylene, pentylene, hexylene, heptylene, octylene, isoprene styrene, and vinylethylene.

Preferred vinyl ethers are divinyl ether, methylvinylether, ethylvinylether, propylvinylether, butylvinylether, pentylvinylether, hexylvinylether, heptylvinylether, and octylvinylether.

Preferred vinyl esters are vinyl formate, vinyl acetate, vinyl propanoate, vinyl butanoate, vinyl pentanoate, vinyl hexanoate, vinyl heptanoate, vinyl octanoate, vinyl nonanoate, vinyl decanoate, vinyl acrylate, vinyl methacrylate, vinyl benzoate, vinyl 4-tert-butylbenzoate, vinyl cinnamate, and vinyl trifluoroacetate.

Preferred polythiols are organosulfur compounds with two or more thiol functional groups. Particularly preferred polythiols are selected from the list consisting of HS-(CₙH₂ₙ)-SH, wherein n = 2 to 20, preferably 2 to 12; CₙH₂ₙ₋₁(SH)₃, wherein n = 3 to 20, preferably 3 to 12; HS-Ar-SH, wherein Ar = substituted or unsubstituted C₆-C₂₀ arylene; and HS-(CH₂)ₘ-Ar-(CH₂)ₘ-SH, wherein Ar = substituted or unsubstituted C₆-C₂₀ arylene and m = 1 to 12.

Preferred polyamines are organoamine compounds with two or more amino functional groups. Particularly preferred polyamines are selected from the list consisting of H₂N-(CₙH₂ₙ)-NH₂, wherein n = 2 to 20, preferably 2 to 12; H₂N-(CₙH₂ₙNH)-NH₂, wherein n = 2 to 20, preferably 2 to 12; CₙH₂ₙ₋₁(NH₂)₃, wherein n = 3 to 20, preferably 3 to 12; H₂N-Ar-NH₂, wherein Ar = substituted or unsubstituted C₆-C₂₀ arylene; and H₂N-(CH₂)ₘ-Ar-(CH₂)ₘ-H₂N, wherein Ar = substituted or unsubstituted C₆-C₂₀ arylene and m = 1 to 12.

Preferred polymaleimides are maleimide end-capped polyimides as described in US 2008/0075961 A1 and US 2017/0152418 A1 the disclosure of which is herewith incorporated by reference. It is preferred that the polymaleimides are dimaleimides selected from compounds represented by the following Formula (A) or Formula (B): 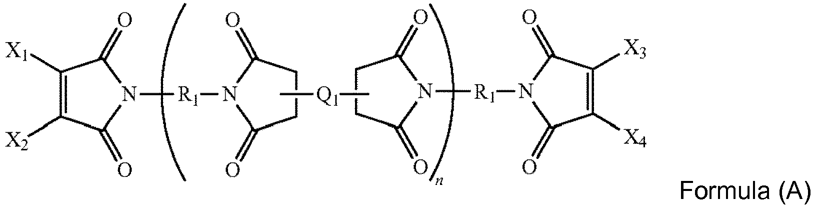
wherein R₁ and Q₁ are independently selected from the group consisting of structures derived from unsubstituted or substituted aliphatic, alicyclic, alkenyl, aryl, heteroaryl, siloxane, poly(butadiene-co-acrylonitrile) and poly(alkylene oxide); X₁ to X₄ are each independently H or an alkyl group with 1 to 6 C atoms; and n = 0 to 30; 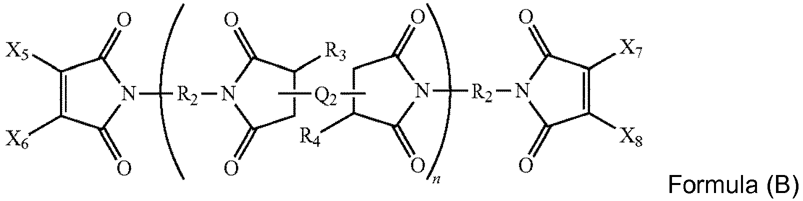
wherein R₂ and Q₂ are independently selected from the group consisting of structures derived from unsubstituted or substituted aliphatic, alicyclic, alkenyl, aryl, heteroaryl, siloxane, poly(butadiene-co-acrylonitrile) and poly(alkylene oxide); X₅ to X₈ are each independently H or an alkyl group with 1 to 6 C atoms; R₃ and R₄ are each independently H or CH₃, wherein at least one of R₃ and R₄ is CH₃; and n = 0 to 30.

In a preferred embodiment, the structure derived from unsubstituted or substituted aliphatic, alicyclic, alkenyl, aryl, heteroaryl, siloxane, poly(butadiene-co-acrylonitrile) and poly(alkylene oxide) are alkyl group, alkenyl group, alkynyl group, hydroxyl group, oxo group, alkoxy group, mercapto group, cycloalkyl group, substituted cycloalkyl group, heterocyclic group, substituted heterocyclic group, aryl group, substituted aryl group, heteroaryl group, substituted heteroaryl group, aryloxy group, substituted aryloxy group, halogen, haloalkyl group, cyano group, nitro group, nitrone group, amino group, amide group, -C(O)H, acyl group, oxyacyl group, carboxyl group, carbamate group, sulfonyl group, sulfonamide group, sulfuryl group, or -C(O)-, -S-, -S(O)₂-, -OC(O)-O-, -NA-C(O)-, -NAC(O)-NA-, -OC(O)-NA-, (in the formula, A is H or an alkyl group with 1 to 6 carbons), and it is preferable that one terminal further contains a substituent.

Preferred substituents are alkyl group, alkenyl group, alkynyl group, hydroxyl group, oxo group, alkoxy group, mercapto group, cycloalkyl group, substituted cycloalkyl group, heterocyclic group, substituted heterocyclic group, aryl group, substituted aryl group, heteroaryl group, substituted heteroaryl group, aryloxy group, substituted aryloxy group, halogen, haloalkyl group, cyano group, nitro group, nitrone group, amino group, amide group, -C(O)H, acyl group, oxyacyl group, carboxyl group, carbamate group, sulfonyl group, sulfonamide group, sulfuryl group, or -C(O)-, -S-, -S(O)₂-, -OC(O)-O-, -NA-C(O)-, -NAC(O)-NA-, -OC(O)-NA-, (in the formula, A is H or an alkyl group with 1 to 6 carbons), acyl group, oxyacyl group, carboxyl group, carbamate group, sulfonyl group, sulfonamide group, or sulfuryl group.

In a more preferred embodiment, R¹ and R², and Q¹ and Q² are independently selected from the group consisting of substituted or unsubstituted aliphatic, alicyclic, alkenyl, aromatic, heteroaromatic, siloxane, poly(butadiene-co-acrylonitrile), or poly(alkylene oxide) moieties.

Preferred aliphatic moieties are straight or branched chain C₁-C₅₀ alkylene, more preferably straight or branched chain C₁-C₃₆ alkylene.

Preferred alicyclic moieties are both aliphatic and cyclic and contain one or more all-carbon rings which may be either substituted or unsubstituted and which may be optionally condensed and/or bridged. Preferred alicyclic moieties have 3 to 72 C atoms, more preferably 3 to 36 C atoms. Particularly preferred alicyclic moieties are represented by -Sp¹-Cy-Sp²-, wherein Sp¹ and Sp² denote independently of each other alkylene having 1 to 12 C atoms or a single bond; G denotes cycloalkylene having 3 to 12 C atoms which is optionally mono- or polysubstituted by alkyl having 1 to 12 C atoms.

Preferred alkenyl moieties are straight or branched chain hydrocarbyl moieties having at least one carbon-carbon double bond, and having in the range of about up to 100 C atoms. More preferred alkenyl moieties are C₂-C₅₀ alkenylene, most preferably C₂-C₃₆ alkenylene.

Preferred aromatic moieties are arylene groups having 6 to 20 C atoms, more preferably 6 to 14 C atoms, which may be either substituted or unsubstituted.

Heteroaromatic moieties are aromatic moieties containing one or more heteroatoms (e.g. N, O, S, or the like) as part of the ring structure. Preferred heteroaromatic moieties have 3 to 20 C atoms, preferably 3 to 14 C atoms, and one or more heteroatoms selected from N, O and/or S, and they may be either substituted or unsubstituted.

Preferred siloxane moieties are selected from -[R^{a}R^{b}Si-O]ₙ-R^{a}R^{b}Si-, wherein R³ and R^{b} are independently H or C₁-C₆ alkyl, and n = 1 to 1000, more preferably 1 to 100.

Preferred poly(alkylene oxide) moieties are poly(C₁-C₁₂ alkylene oxide) moieties.

It is preferred that the molar ratio between the polymerizable compounds of the present invention and the additional polymerizable compounds in the polymerizable starting material is from 0.1:100 to 100:0.1.

It is preferred that the polymerizable starting material provided in step (i) is substantially free of solvent. Substantially free of solvent means that the content of total residual solvent in the polymerizable starting material is not more than 10 wt.-%, preferably not more than 5 wt.-%, and more preferably not more than 1 wt.-%, based on the total weight of the polymerizable compound(s).

It is preferred that the polymerizable starting material is polymerized in step (ii) by radical or ionic polymerization or by exposure to radiation. It is further preferred that the polymerizable starting material contains an initiator for free radical polymerization or an initiator for ionic polymerization. It is further preferred that the polymerization of the polymerizable compounds in step (ii) takes place at elevated temperature, preferably at a temperature in the range from 25 to 200 °C, more preferably at a temperature in the range from 25 to 150 °C.

Preferably, the initiators for radical polymerization are activated thermally by exposure to heat or photochemically by exposure to radiation such as UV and/or visible light.

Preferred initiators for radical polymerization are: *tert-amyl* peroxybenzoate, 4,4-azobis(4-cyanovaleric acid), 1,1'-azobis(cyclohexanecarbonitrile), 2,2'-azobisisobutyronitrile (AIBN), benzoyl peroxide, 2,2-bis(tert-butylperoxy)butane, 1,1-bis(tert-butylperoxy)cyclohexane, 2,5-bis(tert-butylperoxy)-2,5-dimethylhexane, 2,5-bis(tert-butylperoxy)-2,5-dimethyl-3-hexyne, bis(1-(tert-butylperoxy)-1-methylethyl)benzene, 1,1-bis(tert-butylperoxy)-3,3,5-trimethylcyclohexane, tert-butyl hydroperoxide, tert-butyl peracetate, tert-butyl peroxide, tert-butyl peroxybenzoate, tert-butylperoxy isopropyl carbonate, cumene hydroperoxide, cyclohexanone peroxide, dicumyl peroxide, lauroyl peroxide, 2,4-pentanedione peroxide, peracetic acid, and potassium persulfate. Typically, such initiators are radical polymerization initiators which may be thermally activated.

Further preferred initiators for radical polymerization are: acetophenone, *p-*anisil, benzil, benzoin, benzophenone, 2-benzoylbenzoic acid, 4,4'-bis(diethylamino)benzophenone, 4,4'-bis(dimethylamino)benzophenone, benzoin methyl ether, benzoin isopropyl ether, benzoin isobutyl ether, benzoin ethyl ether, 4-benzoylbenzoic acid, 2,2'-bis(2-chlorophenyl)-4,4',5,5'-tetraphenyl-1,2'-biimidazole, methyl 2-benzoylbenzoate, 2-(1,3-benzodioxol-5-yl)-4,6-bis(trichloromethyl)-1,3,5-triazine, 2-benzyl-2-(dimethylamino)-4'-morpholinobutyrophenone, (±)-camphorquinone, 2-chlorothioxanthone, 4,4'-dichlorobenzophenone, 2,2-Diethoxyacetophenone, 2,2-Dimethoxy-2-phenylacetophenone, 2,4-diethylthioxanthen-9-one, diphenyl(2,4,6-trimethylbenzoyl)phosphine oxide, 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isopropylthioxanthone, lithium phenyl(2,4,6-trimethylbenzoyl)phosphinate, 2-methyl-4'-(methylthio)-2-morpholino-propiophenone, 2-isonitrosopropiophenone, 2-phenyl-2-(p-toluenesulfonyl-oxy)acetophenone, and phenylbis(2,4,6-trimethylbenzoyl)phosphine oxide. Typically, such initiators are radical polymerization initiators which may be photochemically activated.

Preferred initiators for ionic polymerization are: alkyl lithium compounds, alkylamine lithium compounds and pentamethylcyclopentadienyl (Cp*) complexes of titanium, zirconium and hafnium.

Further preferred initiators for ionic polymerization are: bis(4-tert-butylphenyl)iodonium hexafluorophosphate, bis(4-fluorophenyl)iodonium trifluoromethanesulfonate, cyclopropyldiphenylsulfonium tetrafluoroborate, dimethylphenacylsulfonium tetrafluoroborate, diphenyliodonium hexafluorophosphate, diphenyliodonium hexafluoroarsenate, diphenyliodonium trifluoromethanesulfonate, 2-(3,4-dimethoxystyryl)-4,6-bis(trichloromethyl)-1,3,5-triazine, 2-[2-(furan-2-yl)vinyl]-4,6-bis(trichloromethyl)-1,3,5-triazine, 4-isopropyl-4'-methyldiphenyliodonium tetrakis(pentafluorophenyl)borate, 2-[2-(5-methylfuran-2-yl)vinyl]-4,6-bis(trichloromethyl)-1,3,5-triazine, 2-(4-methoxyphenyl)-4,6-bis(trichloromethyl)-1,3,5-triazine, 2-(4-methoxystyryl)-4,6-bis(trichloromethyl)-1,3,5-triazine, (2-methylphenyl)(2,4,6-trimethylphenyl)iodonium trifluoromethanesulfonate, (3-methylphenyl)(2,4,6-trimethylphenyl)iodonium trifluoromethanesulfonate, (4-methylphenyl)(2,4,6-trimethylphenyl)iodonium trifluoromethanesulfonate, 4-nitrobenzenediazonium tetrafluoroborate, (4-nitrophenyl)(phenyl)iodonium trifluoromethanesulfonate, triphenylsulfonium tetrafluoroborate, triphenylsulfonium bromide, tri-p-tolylsulfonium hexafluorophosphate, tri-p-tolylsulfonium trifluoromethanesulfonate, [3-(trifluoromethyl)phenyl](2,4,6-trimethylphenyl)iodonium trifluoromethanesulfonate, and [4-(trifluoromethyl)phenyl](2,4,6-trimethylphenyl)iodonium trifluoromethanesulfonate. Typically, such initiators are cationic polymerization initiators which may be photochemically activated.

Further preferred initiators for ionic polymerization are: acetophenone O-benzoyloxime, 1,2-bis(4-methoxyphenyl)-2-oxoethyl cyclohexylcarbamate, nifedipine, 2-nitrobenzyl cyclohexylcarbamate, 2-(9-oxoxanthen-2-yl)propionic acid 1,5,7-triazabicyclo[4.4.0]dec-5-ene salt, 2-(9-oxoxanthen-2-yl)propionic acid 1,5-diazabicyclo[4.3.0]non-5-ene salt, and 2-(9-oxoxanthen-2-yl)propionic acid 1,8-diazabicyclo[5.4.0]undec-7-ene salt. Typically, such initiators are anionic polymerization initiators which may be photochemically activated.

Exposure to radiation includes exposure to visible light and/or UV light. It is preferred that the visible light is electromagnetic radiation with a wavelength from > 380 to 780 nm, more preferably from > 380 to 500 nm. It is preferred that the UV light is electromagnetic radiation with a wavelength of ≤ 380 nm, more preferably a wavelength from 100 to 380 nm. More preferably, the UV light is selected from UV-A light having a wavelength from 315 to 380 nm, UV-B light having a wavelength from 280 to 315 nm, and UV-C light having a wavelength from 100 to 280 nm.

As UV light sources Hg-vapor lamps or UV-lasers are possible, as IR light sources ceramic-emitters or IR-laser diodes are possible and for light in the visible area laser diodes are possible.

Preferred UV light sources are light sources having a) a single wavelength radiation with a maximum of < 255 nm such as e.g. 254 nm and 185 nm Hg low-pressure discharge lamps, 193 nm ArF excimer laser and 172 nm Xe2 layer, or b) broad wavelength distribution radiation with a wavelength component of < 255 m such as e.g. non-doped Hg low-pressure discharge lamps.

In a preferred embodiment of the present invention the light source is a xenon flash light. Preferably, the xenon flash light has a broad emission spectrum with a short wavelength component going down to about 200 nm.

It is preferred that the polymerization in step (ii) takes place in a time range from 0.1 to 1 h, preferably from 0.5 to 1 h.

There is further provided a polymer which is obtainable or obtained by the above-mentioned method for forming a polymer according to the present invention. The polymer is preferably a linear or crosslinked polymer, more preferably a linear polymer.

There is also provided a polymer which contains at least one repeating unit derived from the polymerizable compound of Formula (1) as defined above.

More preferably, said polymer contains repeating units comprising a structural unit represented by the following Formula (4):

[-Sp¹-(MG-Sp¹)ₘ-] Formula (4)

wherein Sp¹, MG and m have one of the above-mentioned definitions.

In a preferred embodiment, the polymer further contains additional repeating units derived from the additional polymerizable compounds as defined above.

Preferably, the polymers according to the present invention have a molecular weight M_{w}, as determined by GPC, of at least 2000 g/mol, more preferably of at least 4000 g/mol, even more preferably of at least 5000 g/mol. Preferably, the molecular weight M_{w} of the polymers is less than 50000 g/mol. More preferably, the molecular weight M_{w} of the polymers is in the range from 5000 to 20000 g/mol.

Moreover there is provided an electronic device comprising a polymer according to the present invention. For the electronic device it is preferred that the polymer forms a dielectric layer. The dielectric layer serves to electrically separate one or more electronic components being part of the electronic device from each other.

Finally, there is provided a manufacturing method for preparing a packaged microelectronic structure, in which a substrate is provided with a dielectric coating comprising one or more polymerized liquid crystalline monomers, wherein the method comprises the following steps:
(1) applying a polymerizable composition comprising one or more polymerizable compounds according to the present invention to a surface of a substrate; and
(2) curing said polymerizable composition to form a dielectric layer.

It is preferred that the polymerizable composition provided in step (1) comprises one polymerizable compound according to the present invention.

In a preferred embodiment, the polymerizable composition provided in step (1) comprises one or more additional polymerizable compounds which are capable to react with the polymerizable compound according to the present invention to form a copolymer.

Preferred additional polymerizable compounds are monomers which are capable to react with the polymerizable group P of the polymerizable compound of the present invention to form a copolymer. Such preferred additional polymerizable compounds are selected from acrylates, epoxides, olefins, vinyl ethers, vinyl esters, polythiols, polyamines, and polymaleimides. Using basic chemical knowledge, the skilled person is able to find and select for a given polymerizable compound of the present invention suitable additional polymerizable compounds which are capable to react with the first mentioned to form a copolymer.

Preferred acrylates, epoxides, olefins, vinyl ethers, vinyl esters, polythiols, polyamines, and polymaleimides are the same as described above.

It is preferred that the molar ratio between the polymerizable compounds of the present invention and the additional polymerizable compounds in the polymerizable composition is from 0.1:100 to 100:0.1.

It is preferred that the polymerizable composition further comprises one or more inorganic filler materials. Preferred inorganic filler materials are selected from nitrides, titanates, diamond, oxides, sulfides, sulfites, sulfates, silicates and carbides which may be optionally surface-modified with a capping agent. More preferably, the filler material is selected from the list consisting of AIN, Al₂O₃, BN, BaTiO₃, B₂O₃, Fe₂O₃, SiO₂, TiO₂, ZrO₂, PbS, SiC, diamond and glass particles.

Preferably, the total content of the inorganic filler material in the polymerizable composition is in the range from 0.001 to 90 wt.-%, more preferably 0.01 to 70 wt.-% and most preferably 0.01 to 50 wt.-%, based on the total weight of the polymerizable composition.

It is preferred that the polymerizable composition provided in step (1) is substantially free of solvent. Substantially free of solvent means that the content of total residual solvent in the polymerizable composition is not more than 10 wt.-%, preferably not more than 5 wt.-%, and more preferably not more than 1 wt.-%, based on the total weight of the polymerizable compound(s).

The method by which the polymerizable composition is applied in step (1) is not particularly limited. Preferred application methods for step (1) are dispensing, dipping, screen printing, stencile printing, roller coating, spray coating, slot coating, spin coating, gravure printing, flexo printing or inkjet printing.

The polymerizable compounds of the present invention may be provided in the form of a formulation suitable for gravure printing, flexo printing and/or ink-jet printing. For the preparation of such formulations, ink base formulations as known from the state of the art can be used.

Alternatively, the polymerizable compound of the present invention may be provided in the form of a formulation suitable for photolithography. The photolithography process allows the creation of a photopattern by using light to transfer a geometric pattern from a photomask to a light-curable composition. Typically, such light-curable composition contains a photochemically activatable polymerization initiator. For the preparation of such formulations, photoresist base formulations as known from the state of the art can be used.

The layer of the polymerizable composition which is applied in step (1) has preferably an average thickness of 1 to 50 µm, more preferably 2 to 30 µm, and most preferably 3 to 15 µm.

It is preferred that the curing in step (2) is carried out by radical or ionic polymerization or by exposure to radiation. Preferred curing conditions correspond to the preferred polymerization conditions as given above for the method for forming a polymer material.

There is also provided a microelectronic device which comprises the packaged microelectronic structure prepared according to the above-mentioned manufacturing method.

The present invention is further illustrated by the examples following hereinafter which shall in no way be construed as limiting. The skilled person will acknowledge that various modifications, additions and alternations may be made to the invention without departing from the spirit and scope of the invention as defined in the appended claims.

### Examples

### A Synthesis of Dianhydrides

1. Synthesis of 2-methyl-1,4-phenylene bis(4-hydroxybenzoate) (1) 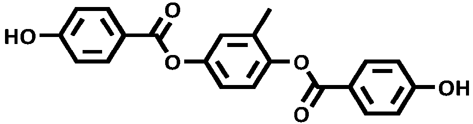 A mixture of 4-hydroxybenzoic acid (111 g, 0.8 mol), 2-methylhydroquinone (50 g, 0.4 mol), *p*-toluenesulfonic acid monohydrate (7.7 g, 0.04 mol) and *p-*xylene (400 mL) was heated to reflux using a Dean-Stark apparatus for 20 h. The reaction mixture was cooled to room temperature and the product was isolated by filtration, washed with methanol (400 mL) and vacuum-dried at 40 °C for 12 h to give the product as a white crystalline solid (136 g, 93%). LC-MS (ESI-MS): m/z = 365 g/mol [M+1H]¹⁺.
2. Synthesis of (((2-methyl-1,4-phenylene)bis(oxy))bis(carbonyl)bis (4,1-phenylene) bis(1,3-dioxo-1,3-dihydroisobenzofuran-5-carboxylate (2) 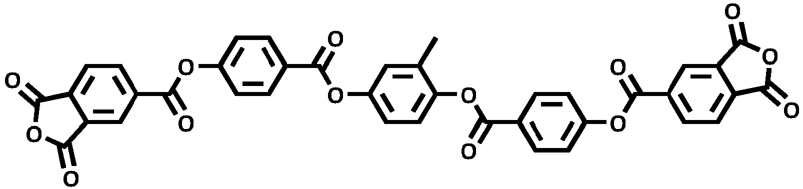 Diphenol 1 (72 g, 0.2 mol) was dissolved in dry THF (1.2 L) in the presence of pyridine (50 mL, 0.62 mol) as an HCl acceptor. In a separated flask trimellitic anhydride chloride (TMAC, 85 g, 0.41 mol) was dissolved in dry THF (0.8 L). The diphenol solution was slowly added to the TMAC solution at 0 °C. After stirring for 20 h at room temperature the formed precipitate was filtered, washed with THF, water and again THF and vacuum-dried at 80 °C for 12 h. An excess of Ac₂O was added to the partially hydrolyzed product and heated at 120 °C for 3 h to ensure cyclodehydration. Ac₂O was azeotropically removed with toluene. The product was finally purified by recrystallization from toluene and vacuum-dried at 160 °C for 12 h (104 g, 71 %). ¹H-NMR (500 MHz, DMSO-*d*₆): δ = 8.74 - 8.62 (m, 3H), 8.41 - 8.22 (m, 6H), 7.71 - 7.66 (m, 3H), 7.65 - 7.61 (m, 1H), 7.40 - 7.36 (m, 3H), 7.28 (dd, *J* = 8.6, 2.7 Hz, 1H), 2.25 (s, 3H) ppm.
3. Synthesis of 2-methyl-1,4-phenylene bis(1,3-dioxo-1,3-dihydroiso-benzofuran-5-carboxylate) (3) 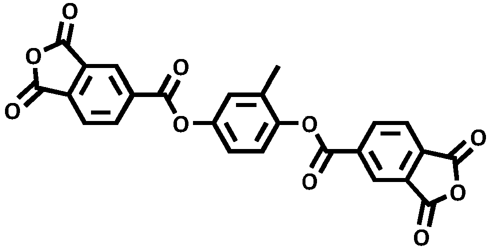 2-Methyl hydroquinone (30 g, 0.24 mol) was dissolved in a mixed solvent of dry THF (0.4 L) and pyridine (59 mL, 0.73 mol). Trimellitic anhydride chloride (104 g, 0.48 mol) was dissolved in anhydrous THF (0.3 L) and added dropwise to the methyl hydroquinone solution at 0 °C with continuous stirring over 1 h, then subsequently reacted at room temperature for 12 h. The reaction mixture was filtered off to collect the white precipitate consisting of crude product and pyridine/hydrochloride salt as by-product. The precipitate was repeatedly washed with water and THF to remove the salt and subsequently dried at 160 °C for 24 h in vacuum. Since the product undergoes partial hydrolysis toward anhydride ring opening on the procedure, the solid was suspended in dry toluene (600 mL), added with DMF (6 drops) and oxalyl chloride (49.5 g, 0.39 mol) and stirred at reflux for 8 h to ensure ring closure dehydration. The batch is cooled to room temperature, filtered and washed with dry toluene and dry THF and finally dried at 120 °C in vacuum. The crude product obtained was finally recrystallized from a mixed solvent of 1,4-dioxane/THF (1/1, v/v) then vacuum-dried again at 160 °C for 24 h to yield the product as a white solid (102 g, 89 %). ¹H NMR (500 MHz, DMSO-*d*₆): δ = 8.71 (dd, *J* = 7.9, 1.4 Hz, 1H), 8.69 - 8.65 (m, 2H), 8.64 (t, *J* = 1.1 Hz, 1H), 8.31 (ddd, *J* = 7.9, 4.6, 0.8 Hz, 2H), 7.47 (d, *J* = 8.8 Hz, 1H), 7.45 (dd, *J* = 2.8, 0.8 Hz, 1H), 7.37 (dd, *J* = 8.6, 2.9 Hz, 1H), 2.26 (s, 3H) ppm.
4. Synthesis of [1,1'-biphenyl]-4,4'-diyl bis(1,3-dioxo-1,3-dihydroiso-benzo-furan-5-carboxylate (4) 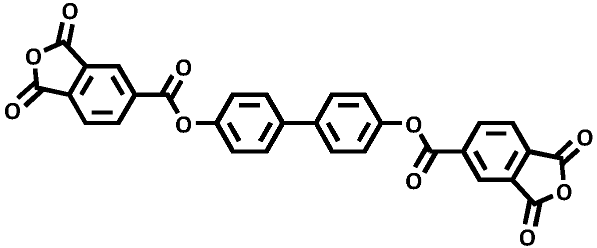 4,4'-Dihydroxybiphenyl (60 g, 0.32 mol) was dissolved in a mixed solvent of dry DMF (0.6 L) and pyridine (80 mL, 0.97 mol). Trimellitic anhydride chloride (139 g, 0.64 mol) was dissolved in anhydrous DMF (0.4 L) and added dropwise to the 4,4'-dihydroxybiphenyl solution at 0 °C with continuous stirring over 1 h, then subsequently reacted at 80 °C for 20 h. The reaction mixture was filtered off to collect the yellow precipitate consisting of crude product and pyridine/hydrochloride salt as by-product. The precipitate was repeatedly washed with water and dry THF to remove the salt. Since the product undergoes partial hydrolysis toward anhydride ring opening on the procedure, it was heated at 160 °C for 24 h in vacuum to ensure ring closure dehydration to yield the product as a yellow solid (138 g, 80 %). ¹H-NMR (500 MHz, DMSO-*d*₆): δ = 8.72 - 8.64 (m, 4H), 8.31 (d, *J* = 8.6, 2H), 7.86 (d, *J* = 8.3 Hz, 4H), 7.53 (J = 8.3 Hz, 4H) ppm.
5. Synthesis of 1,4-phenylene bis(4-hydroxybenzoate) (5) 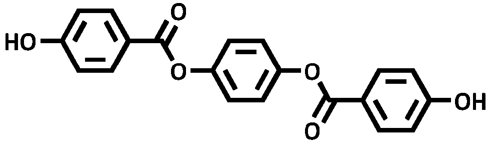 A mixture of 4-hydroxybenzoic acid (49 g, 0.35 mol), hydroquinone (20 g, 0.176 mol), *p*-toluenesulfonic acid monohydrate (3.4 g, 0.017 mol) and *p-*xylene (0.2 L) was heated to reflux using a Dean-Stark apparatus for 20 h. The reaction mixture was cooled to room temperature and the product was isolated by filtration, washed with methanol (400 mL) and vacuum-dried at 40 °C for 12 h to yield the product as a white crystalline solid (59 g, 95%). ¹H-NMR (500 MHz, DMSO-*d*₆): δ = 10.54 (s, 2H), 8.04 - 7.97 (m, 4H), 7.33 (s, 4H), 6.98 - 6.91 (m, 4H) ppm. LC-MS (ESI-MS): m/z = 351 g/mol [M+1 H]¹⁺.
6. Synthesis of ((1,4-phenylenebis(oxy))bis(carbonyl))bis (4,1-phenylene) bis(1,3-dioxo-1,3-dihydroisobenzofuran-5-carboxylate) (6) 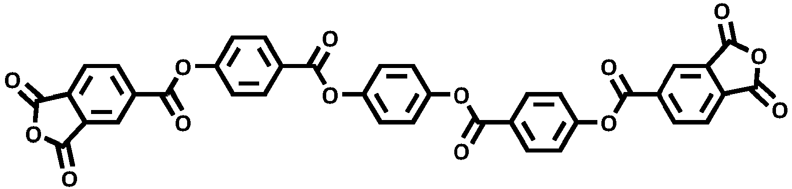 Trimellitic anhydride chloride (TMAC, 147 g, 0.685 mol) was dissolved in dry THF (1.0 L) in the presence of pyridine (50.8 g, 0.64 mol). In a separated flask diphenol 5 (75 g, 0.21 mol) was dissolved in dry THF (0.8 L) and slowly added to the TMAC solution at 0 °C. After stirring for 20 h at reflux the formed precipitate was filtered, washed with THF, water and again THF and vacuum-dried at 160 °C for 20 h (104 g, 71 %). ¹H-NMR (500 MHz, DMSO-*d*₆): δ = 8.74 - 8.62 (m, 3H), 8.36 - 8.27 (m, 6H), 8.22 (dd, J = 8.9, 2.9 Hz, 2H), 7.68 (dd, J = 8.8, 2.4 Hz, 2H), 7.50 - 7.35 (m, 5H) ppm.
7. Synthesis of 4,4'-((1s,3s,5r,7r)-adamantane-1,3-diyl)diphenol (7) 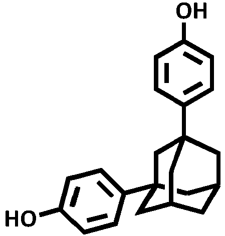 Phenol (47 g, 0.5 mol, dissolved in 920 mL TFA) was treated with 1,3-adamantanediol (40 g, 0.24 mol) and heated at reflux for 20 h. The reaction mixture was cooled to room temperature and quenched cautiously with H₂O (1 L). A white solid precipitated, which was suspended in DCM and stirred overnight at 35 °C. The solid was filtered off, washed with DCM and dried under vacuum to yield 58 g (76 %) of white crystalline solid.
   ¹H-NMR (500 MHz, DMSO-*_{d6}*) δ = 8.99 (broad s), 7.20 - 7.16 (m, 4H), 6.72 - 6.67 (m, 4H), 2.21 (p, *J =* 3.0, 2H), 1.87 - 1.79 (m, 10H), 1.71 (d, *J* = 3.1 Hz, 2H) ppm.
8. Synthesis of ((1s,3s,5r,7r)-adamantane-1,3-diyl)bis(4,1-phenylene) bis(1,3-dioxo-1,3-dihydroisobenzofuran-5-carboxylate) (8) 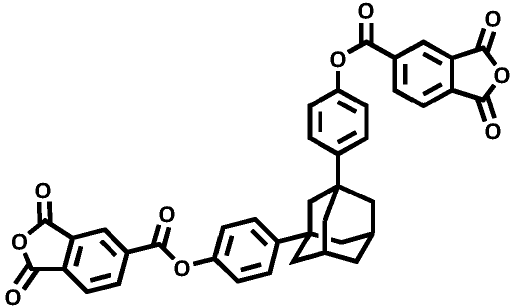 Trimellitic anhydride chloride (TMAC, 43 g, 0.2 mol) was dissolved in dry THF (0.3 L) in the presence of pyridine (14.8 g, 0.19 mol). In a separated flask diphenol 7 (20 g, 0.062 mol) was dissolved in dry THF (0.3 L) and slowly added to the TMAC solution at 0 °C. After stirring for 20 h at reflux the formed precipitate was filtered, washed with THF, water and again THF and vacuum-dried at 45 °C for 20 h. An excess of Ac₂O was added to the partially hydrolyzed product and heated at 120 °C for 12 h to ensure cyclodehydration. The reaction mixture was filtered, washed with THF and vacuum-dried at 100 °C for 12 h (29.6 g, 71 % of a lightly yellow solid). ¹H-NMR (500 MHz, DMSO-*d*₆): δ = 8.67 - 8.59 (m, 4H), 8.28 (d, *J* = 7.9 Hz, 2H), 7.58 (d, *J* = 8.4 Hz, 4H), 7.33 (d, *J* = 8.2 Hz, 4H), 2.31 (s, 2H), 2.06 (s, 2H), 2.02 - 1.91 (m, 8H), 1.79 (s, 2H) ppm.

### B Synthesis of Oligomers

### 9. Example 1

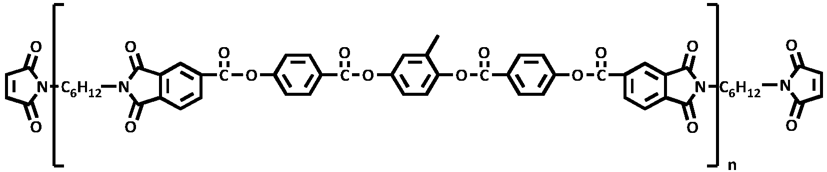

Dianhydride 2 (30 g, 0.04 mol) was dissolved in NMP/toluene (0.3 L, 1/1, v/v). A solution of hexamethylenediamine (9.8 g, 0.08 mol) in NMP (50 mL) was added and the reaction mixture was heated to reflux using a Dean-Stark apparatus for 12 h. The reaction mixture was cooled to room temperature and maleic anhydride (9.8 g, 0.1 mol) was slowly added. The mixture was heated again to reflux using a Dean-Stark apparatus for 12 h. An additional 100 mL of toluene was added after the mixture had been cooled down to room temperature. The mixture was finally added drop-wise to methanol to precipitate the oligomer. After filtering and drying 11.7 g (85%) of the oligomer was yielded as brown powder.

### 10. Example 2

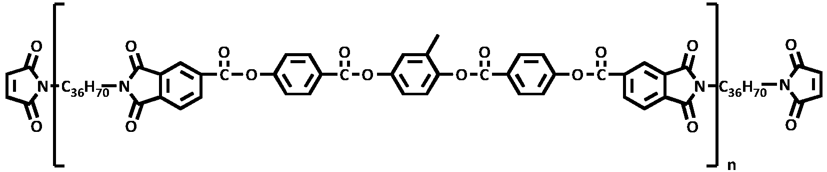

Dianhydride 2 (30 g, 0.04 mol) was dissolved in NMP/toluene (0.3 L, 1/1, v/v) followed by the addition of priamine (45 g, 0.08 mol). The reaction mixture was heated to reflux using a Dean-Stark apparatus for 12 h. The reaction mixture was cooled to room temperature and maleic anhydride (9.8 g, 0.1 mol) was slowly added. The mixture was heated again to reflux using a Dean-Stark apparatus for 12 h. An additional 50 mL of toluene was added after the mixture had been cooled down to room temperature. The mixture was finally added drop-wise to methanol (0.6 L) to precipitate the oligomer. After removing the solvent the crude product was washed twice with methanol and vacuum-dried to produce 65 g (81 %) of a brown waxy resin.

### 11. Example 3

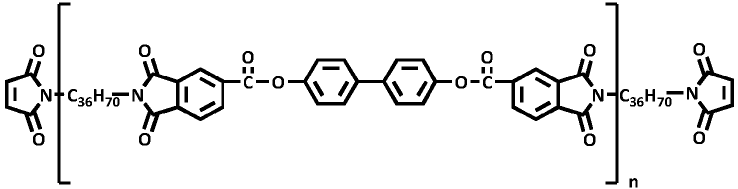

Dianhydride 4 (21 g, 0.04 mol) was suspended in NMP/toluene (0.3 L, 1/1, v/v) followed by the addition of priamine (45 g, 0.08 mol). The reaction mixture was heated to reflux using a Dean-Stark apparatus for 12 h. The reaction mixture was cooled to room temperature and maleic anhydride (10 g, 0.1 mol) was slowly added. The mixture was heated again to reflux using a Dean-Stark apparatus for 12 h. An additional 50 mL of toluene was added after the mixture had been cooled down to room temperature. The mixture was finally added drop-wise to methanol (0.6 L) to precipitate the oligomer. After removing the solvent the crude product was washed twice with methanol and vacuum-dried to produce 61 g (88 %) of a dark waxy resin.

### 12. Example 4

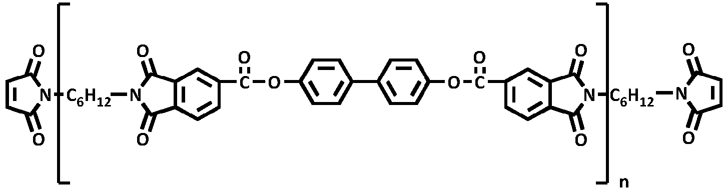

Dianhydride 4 (21 g, 0.04 mol) was dissolved in NMP/toluene (0.3 L, 1/1, v/v). A solution of hexamethylenediamine (9.8 g, 0.08 mol) in NMP (50 mL) was added and the reaction mixture was heated to reflux using a Dean-Stark apparatus for 12 h. The reaction mixture was cooled to room temperature and maleic anhydride (9.8 g, 0.1 mol) was slowly added. The mixture was heated again to reflux using a Dean-Stark apparatus for 12 h. An additional 100 mL of toluene was added after the mixture had been cooled down to room temperature. The mixture was finally added drop-wise to methanol to precipitate the oligomer. After filtering and drying 25 g (70%) of the oligomer was yielded as brown powder.

### 13. Example 5

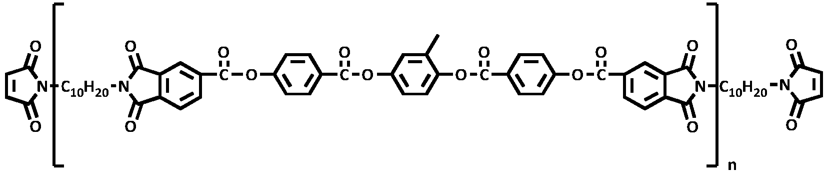

Dianhydride 2 (15 g, 0.02 mol) was suspended in NMP/toluene (0.2 L, 1/1, v/v) followed by the addition of decane-1,10-diamine (7.3 g, 0.04 mol). The reaction mixture was heated to reflux using a Dean-Stark apparatus for 12 h. The reaction mixture was cooled to room temperature and maleic anhydride (5.2 g, 0.052 mol) was slowly added. The mixture was heated again to reflux using a Dean-Stark apparatus for 12 h. An additional 0.2 L of toluene was added after the mixture had been cooled down to room temperature. The mixture was finally added drop-wise to methanol (0.8 L) to precipitate the oligomer. After removing the solvent the crude product was washed twice with methanol and vacuum-dried to produce 24 g (96 %) of a brown solid.

### 14. Example 6

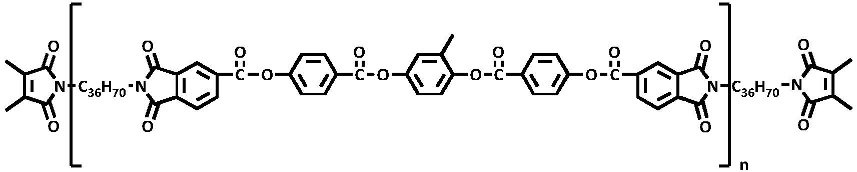

Dianhydride 2 (30 g, 0.04 mol) was dissolved in NMP/toluene (0.3 L, 1/1, v/v) followed by the addition of priamine (45 g, 0.08 mol). The reaction mixture was heated to reflux using a Dean-Stark apparatus for 12 h. The reaction mixture was cooled to room temperature and 2,3-dimethylmaleic anhydride (13.7 g, 0.1 mol) was slowly added. The mixture was heated again to reflux using a Dean-Stark apparatus for 12 h. An additional 100 mL of toluene was added after the mixture had been cooled down to room temperature. The mixture was finally added drop-wise to methanol (0.5 L) to precipitate the oligomer. After removing the solvent the crude product was washed twice with methanol and vacuum-dried to produce 54 g (65 %) of a brown waxy resin.

### 15. Example 7

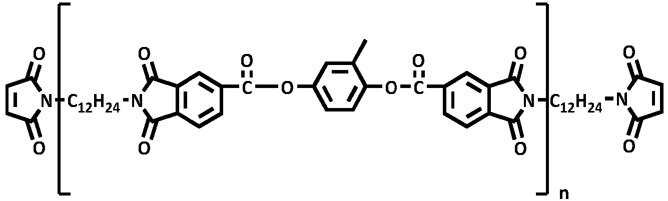

Dianhydride 3 (13 g, 0.028 mol) was suspended in NMP/toluene (0.3 L, 1/1, v/v) followed by the addition of 1,12-diaminododecane (11.5 g, 0.056 mol). The reaction mixture was heated to reflux using a Dean-Stark apparatus for 12 h. The reaction mixture was cooled to room temperature and maleic anhydride (7.0 g, 0.07 mol) was slowly added. The mixture was heated again to reflux using a Dean-Stark apparatus for 12 h. An additional 0.2 L of toluene was added after the mixture had been cooled down to room temperature. The mixture was finally added drop-wise to methanol (0.8 L) to precipitate the oligomer. After removing the solvent the crude product was washed twice with methanol and vacuum-dried to produce 20 g (71 %) of a brown solid.

### 16. Example 8

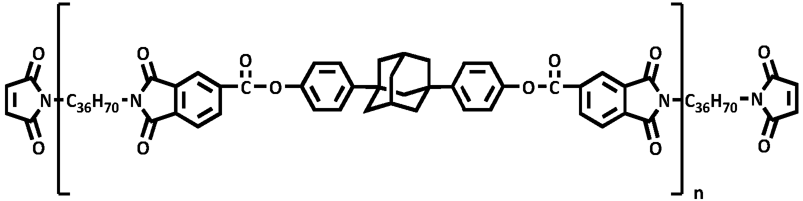

Dianhydride 8 (10 g, 0.015 mol) was dissolved in NMP/toluene (0.1 L, 1/1, v/v) followed by the addition of priamine (16.5 g, 0.03 mol). The reaction mixture was heated to reflux using a Dean-Stark apparatus for 12 h. The reaction mixture was cooled to room temperature and maleic anhydride (3.7 g, 0.04 mol) was slowly added. The mixture was heated again to reflux using a Dean-Stark apparatus for 12 h. An additional 50 mL of toluene was added after the mixture had been cooled down to room temperature. The mixture was finally added drop-wise to methanol (0.2 L) to precipitate the oligomer. After removing the solvent the crude product was washed twice with methanol and vacuum-dried to produce 20 g (71 %) of a brown waxy resin.

### C Mechanical Testing

### 17. Example: Tensile strength and elongation at break

Tensile strength and elongation at break were measured from some of the example polymer specimens (film dimension 25 mm long, 15 mm wide, typically 50-60 µm thick) on a mechanical testing machine (500 N Zwicki) (see Table 1). The measurements were performed according to the following parameter set: premeasurement: 0.1 N at an extension rate of 10 mm/min; main extension rate of 50 mm/min. All experiments were conducted at room temperature (23 ± 2 °C).

**Table 1: Measurement of elongation at break and tensile strength.**

| | **UV dose [min]** | **Elongation at break [%]** | **Tensile strength [MPa]** |
|---|---|---|---|
| **Example 2** | | | |
| Mₙ = 9.600 Da | 0.5 | 245 | 6 |
| | 3 | 206 | 17 |
| Mₙ = 14.000 Da | 0.5 | 231 | 22 |
| | 3 | 157 | 19 |

| **Example 6** | | | |
|---|---|---|---|
| Mₙ = 7.000 Da | 0.5 | 255 | 4 |
| | 3 | 209 | 8 |
| Mₙ = 15.000 Da | 0.5 | 288 | 24 |
| | 3 | 242 | 14 |

### D Electrical Properties

### 18. Example: Evaluation of the dielectric constant

ITO (indium tin oxide) glass was coated via spin coating process with a defined thickness of the materials. The thickness was measured with a profilometer from Bruker. Subsequently, a silver layer was vacuum deposited via a mask process to avoid short cuts on the coated area. The resistance of the layer stacks was determined with an LCR meter and the dielectric constant was calculated. The results are shown in Table 2.

**Table 2: Calculation of dielectric constants**

| **Material** | **Layer thickness [µm]** | **Capacitance [nF]** | **Dielectric constant** |
|---|---|---|---|
| Example 3 (a) | 3.66 | 0.17 | 3.6 |
| Example 3 (b) | 3.18 | 0.2 | 3.7 |
| Example 3 (c) | 2.99 | 0.21 | 3.6 |
| Example 6 (a) | 3.21 | 0.21 | 3.9 |
| Example 6 (b) | 3.23 | 0.19 | 3.5 |
| Example 6 (c) | 4.04 | 0.17 | 4.0 |

## Claims

1. Polymerizable compound selected from 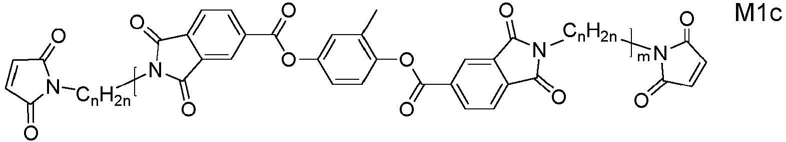 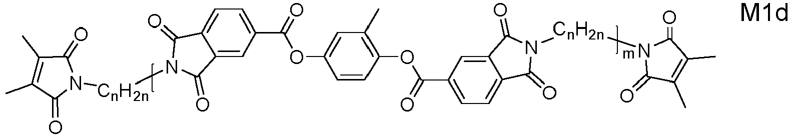 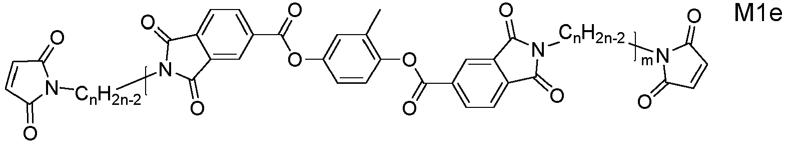 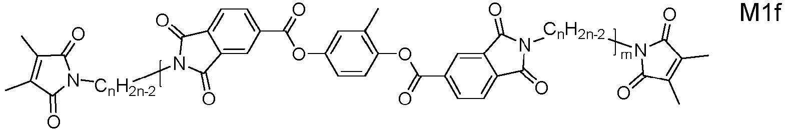 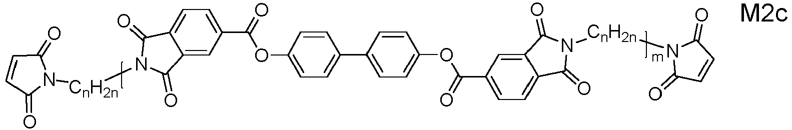 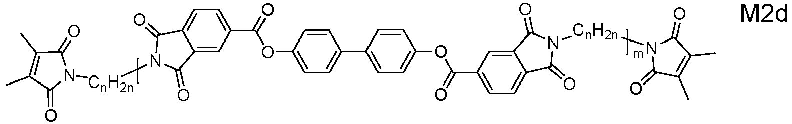 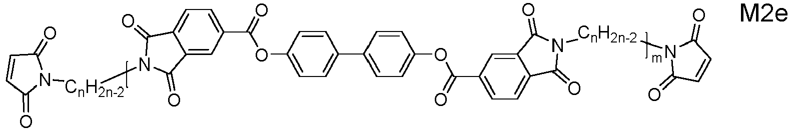 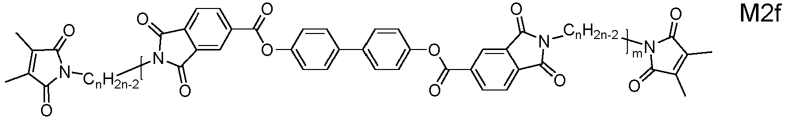 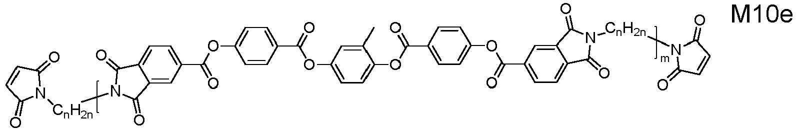 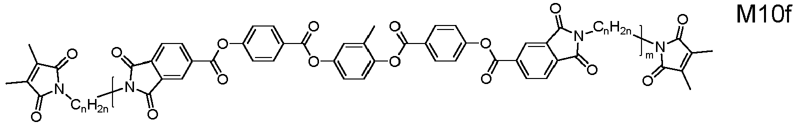 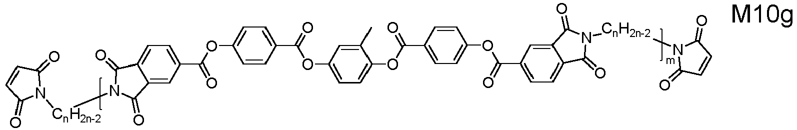 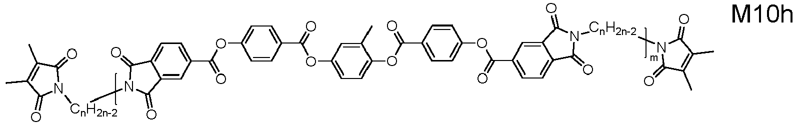 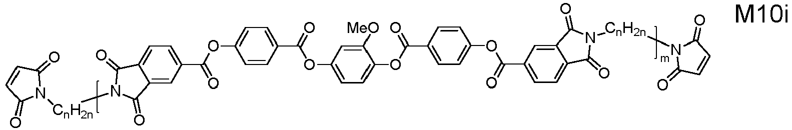 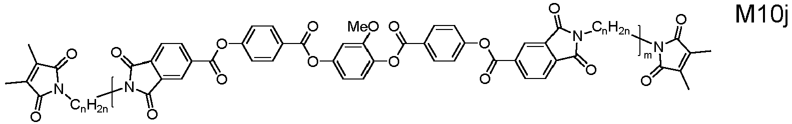 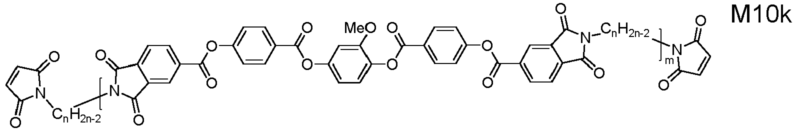 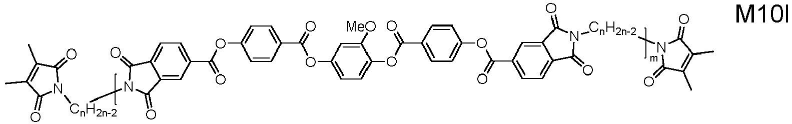 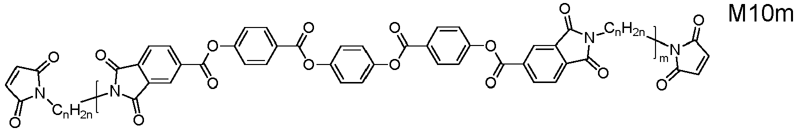 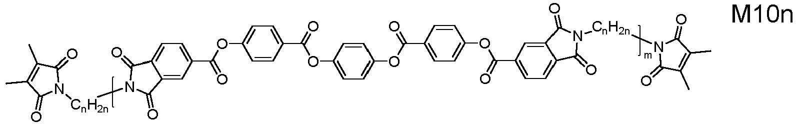 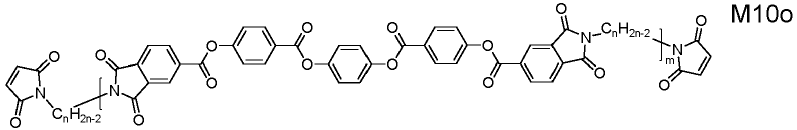 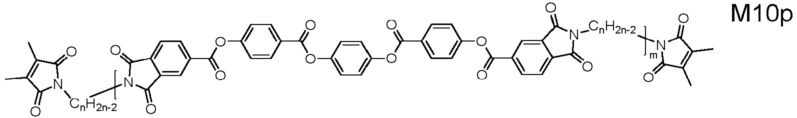 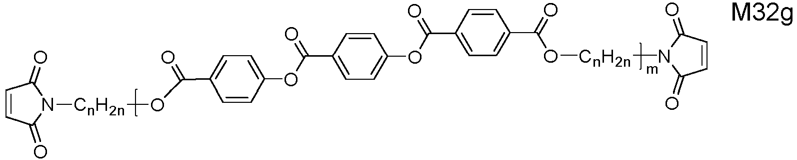 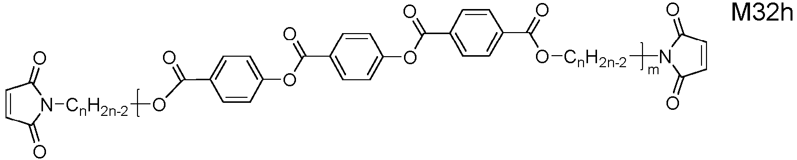 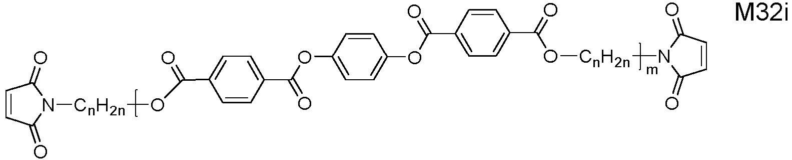 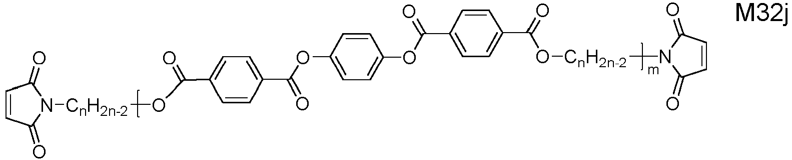 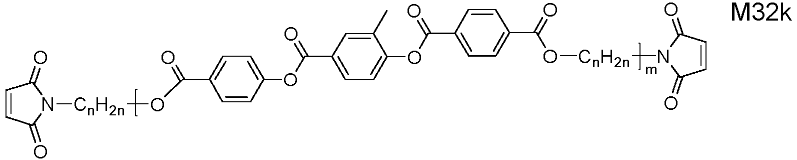 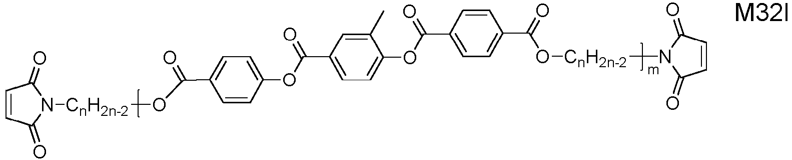 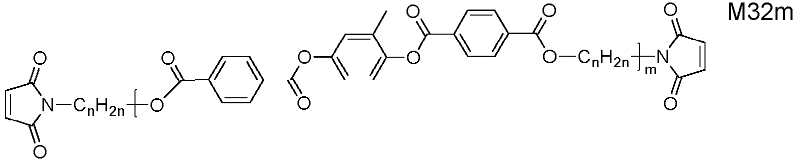 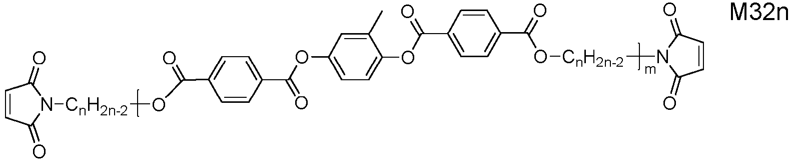 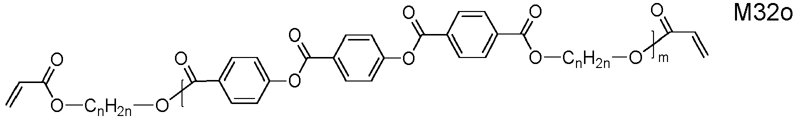 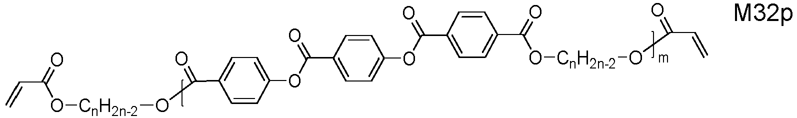 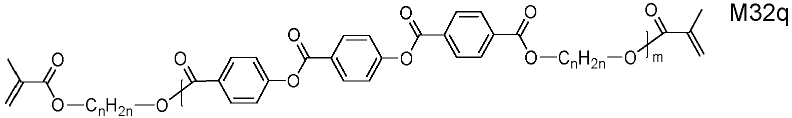 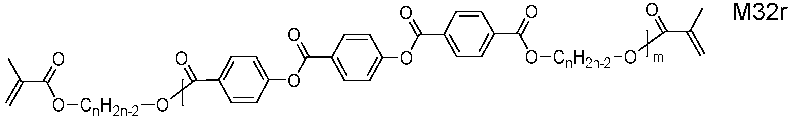 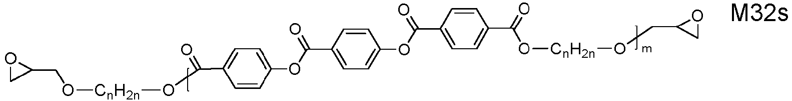 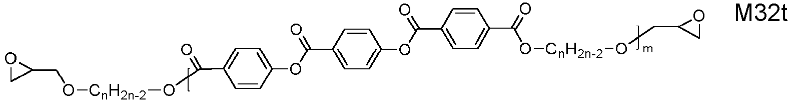 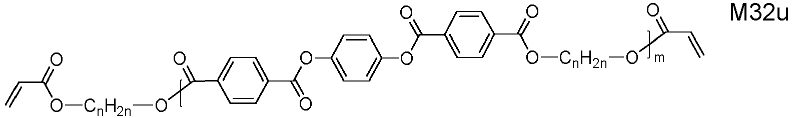 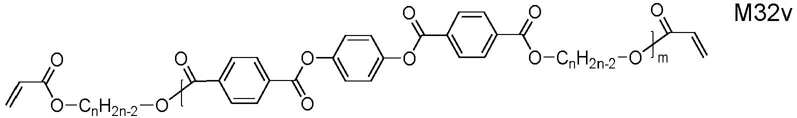 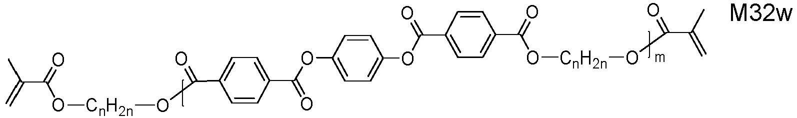 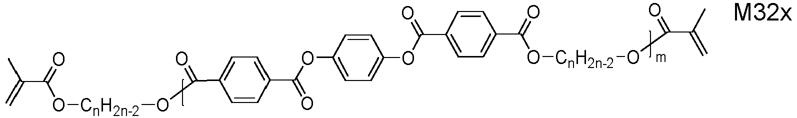 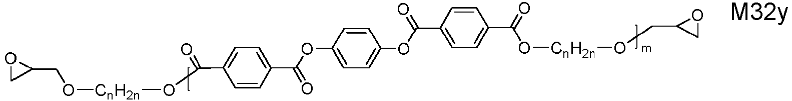 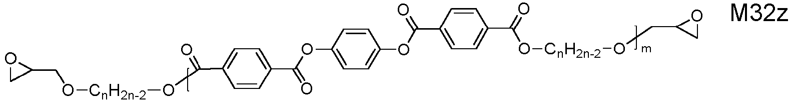 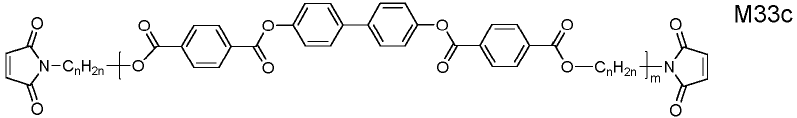 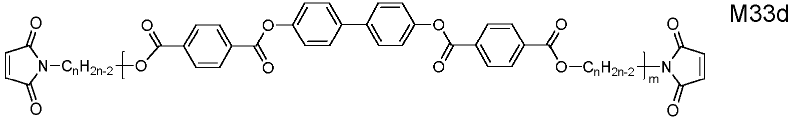 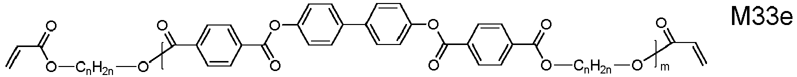 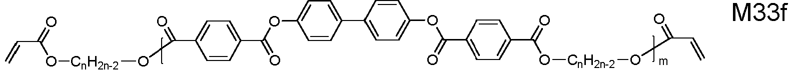 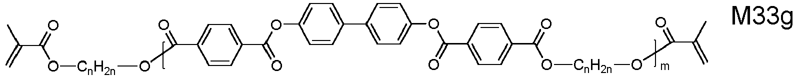 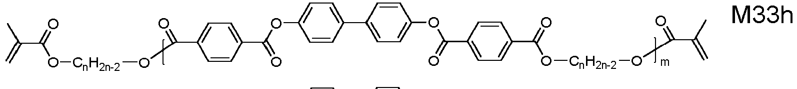 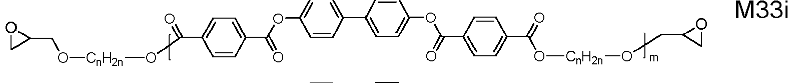 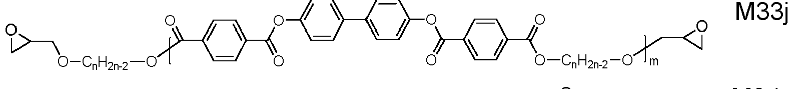 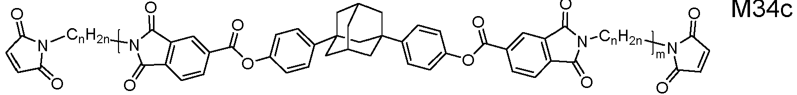 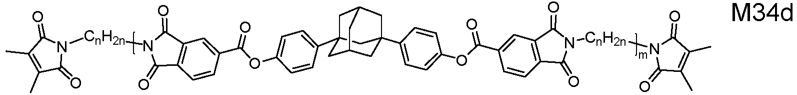 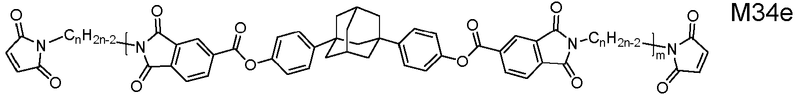 or 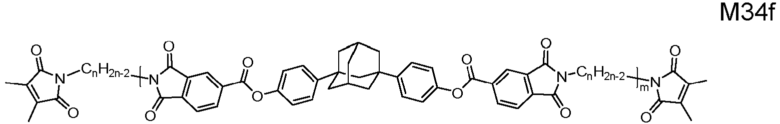 wherein
n is an integer from 1 to 60, preferably from 1 to 36, and more preferably from 6 to 12; and
m is an integer from 1 to 60, preferably 1 to 50, more preferably 2 to 30, and most preferably 3 to 20.

2. Polymerizable compound of claim 1 is selected from M34c, M34d, M34e or M34f

3. Polymerizable compound of claim 1 is

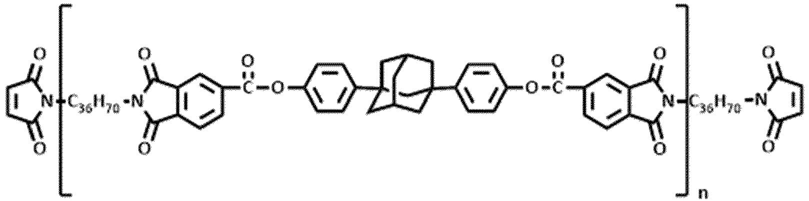

4. Method for forming a polymer comprising the following steps:
(i) providing a polymerizable starting material comprising one or more polymerizable compounds any one of claims 1 to 3; and
(ii) polymerizing said polymerizable starting material.

5. Method for forming a polymer according to claim 4, wherein the polymerizable starting material further comprises one or more additional polymerizable compounds which are capable to react with the polymerizable compound to form a copolymer.

6. Method for forming a polymer according to claim 4 or 5, wherein the polymerizable starting material is polymerized in step (ii) by radical or ionic polymerization or by exposure to radiation.

7. Polymer, obtainable by the method for forming a polymer according to any one of claims 4 to 6.

8. Electronic device comprising a polymer according to claim 7.

9. Electronic device according to claim 8, wherein the polymer forms a dielectric layer.

10. Manufacturing method for preparing a packaged microelectronic structure, in which a substrate is provided with a coating comprising one or more polymerized liquid crystalline monomers, wherein the method comprises the following steps:
(1) applying a polymerizable composition comprising one or more polymerizable compounds any one of claims 1 to 3 to a surface of a substrate; and
(2) curing said polymerizable composition to form a dielectric layer.

11. Manufacturing method for preparing a packaged microelectronic structure according to claim 10, wherein the polymerizable composition further comprises one or more additional polymerizable compounds which are capable to react with the polymerizable compound to form a copolymer.

12. Manufacturing method for preparing a packaged microelectronic structure according to claim 10 or 11, wherein the polymerizable composition further comprises one or more inorganic filler materials.

13. Microelectronic device comprising a packaged microelectronic structure obtainable by the manufacturing method according to any one of claims 10 to 12.

## Patentansprüche

1. Polymerisierbare Verbindung ausgewählt aus 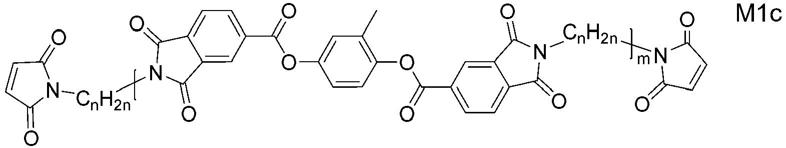 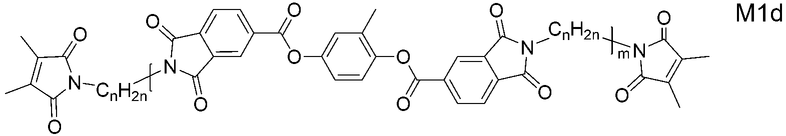 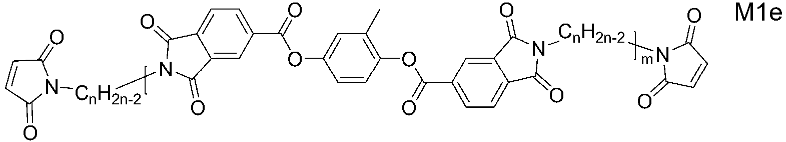 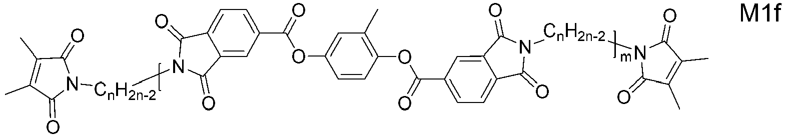 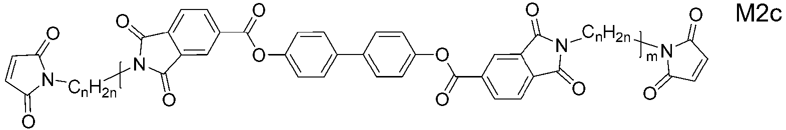 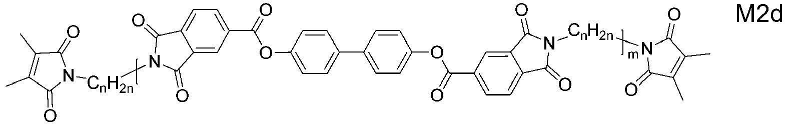 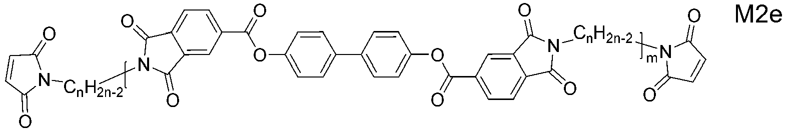 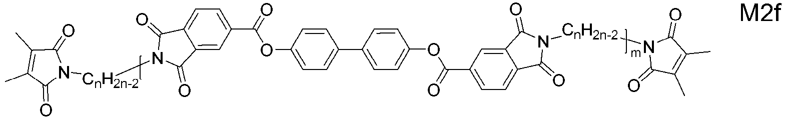 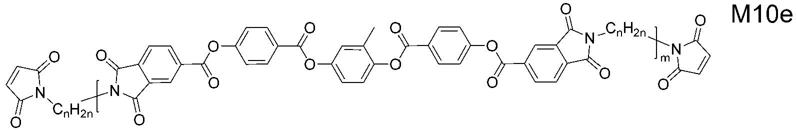 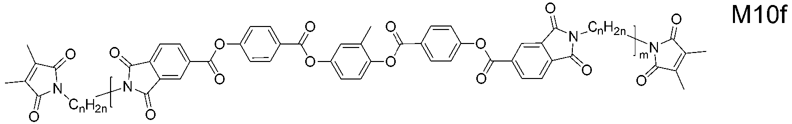 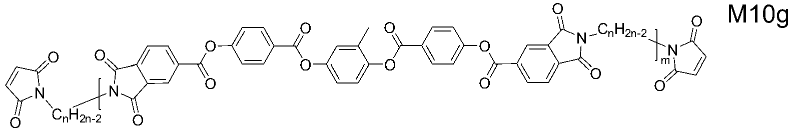 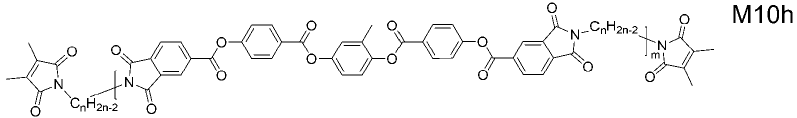 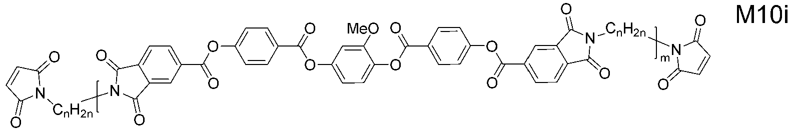 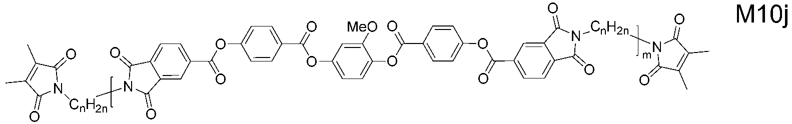 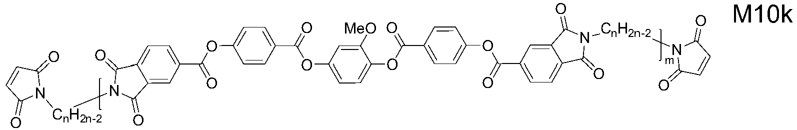 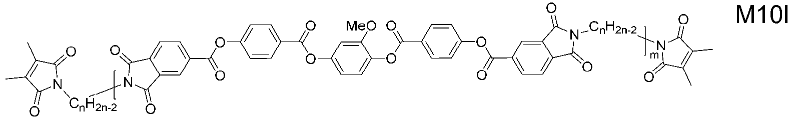 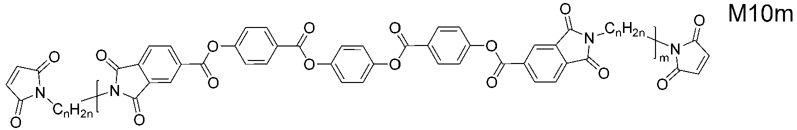 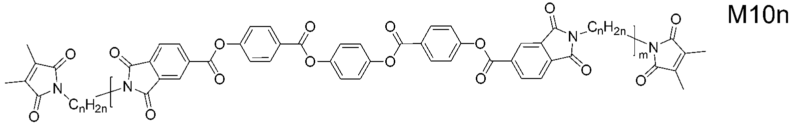 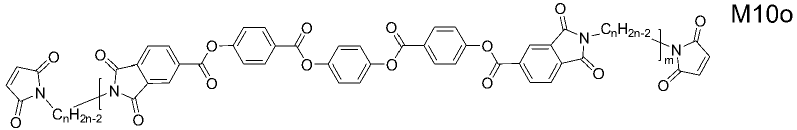 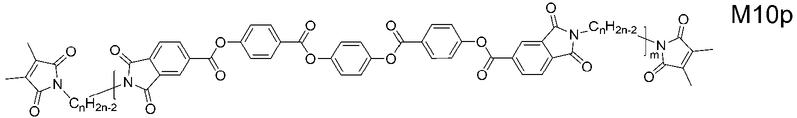 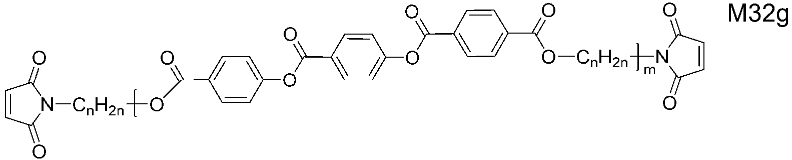 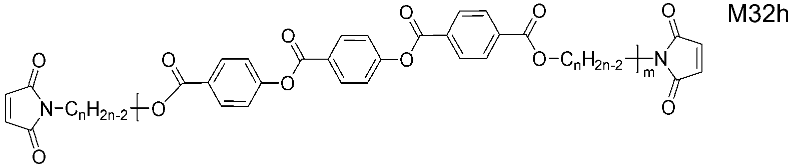 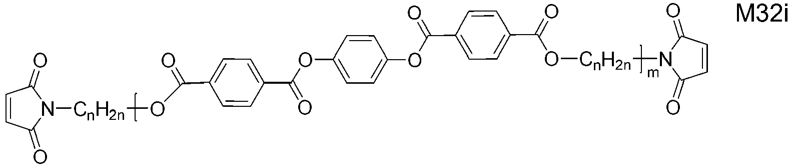 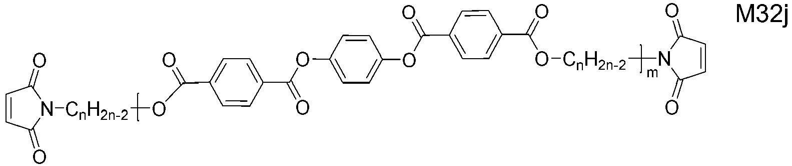 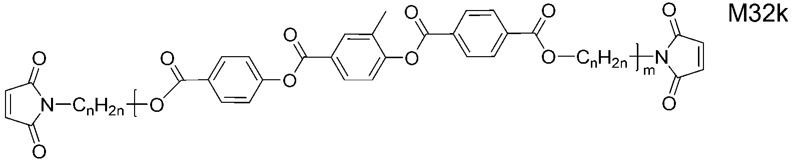 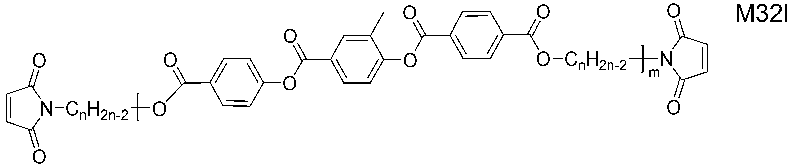 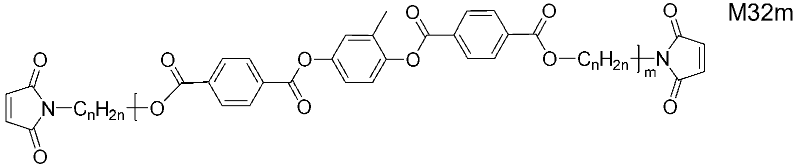 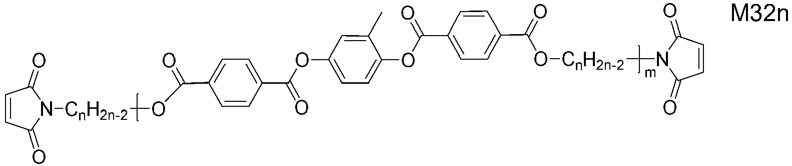 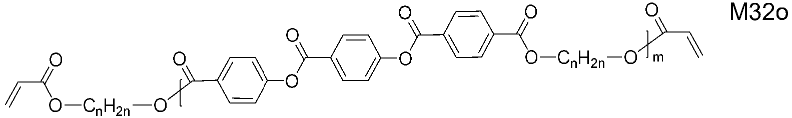 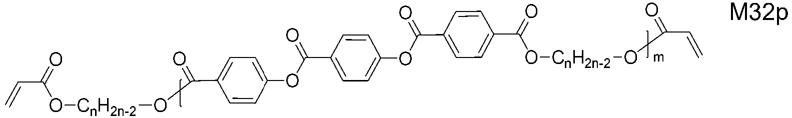 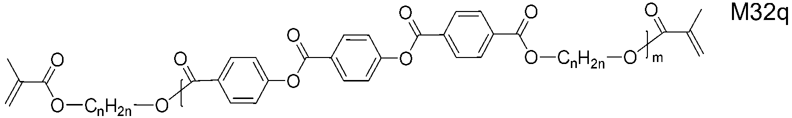 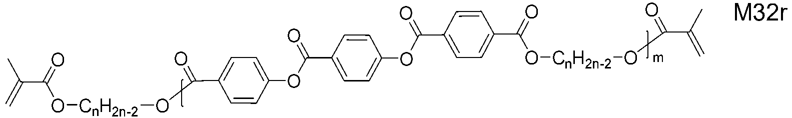 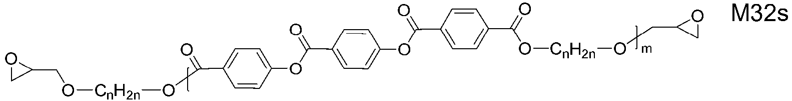 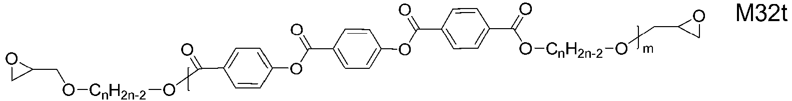 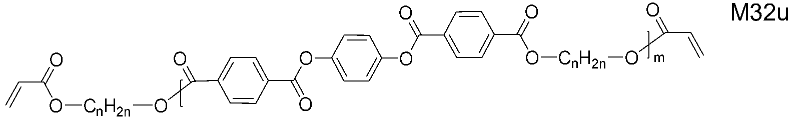 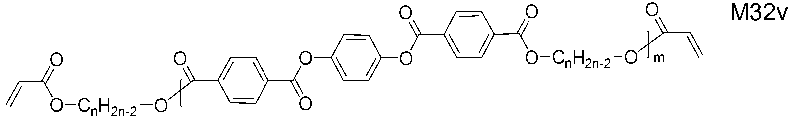 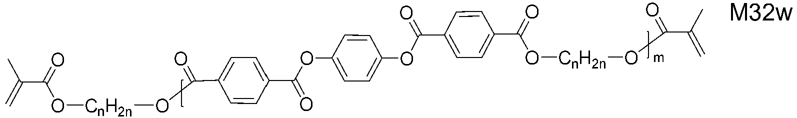 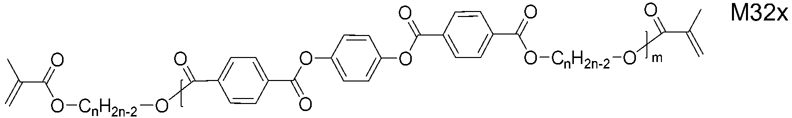 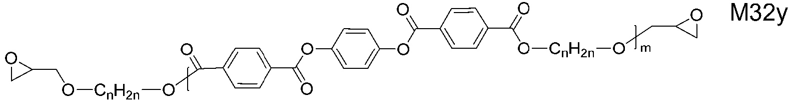 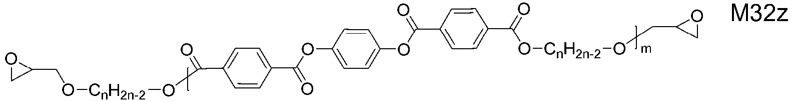 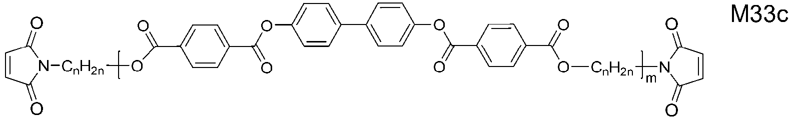 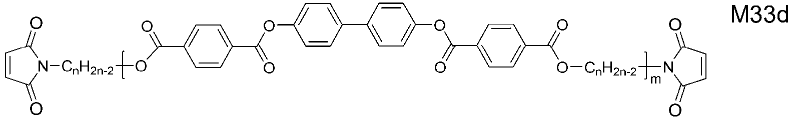 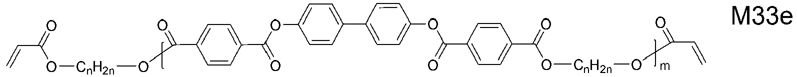 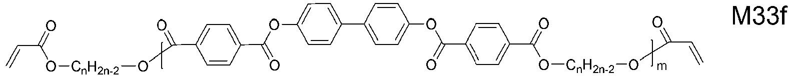 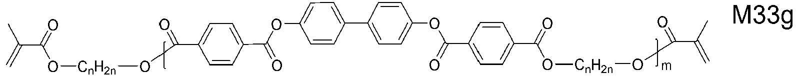 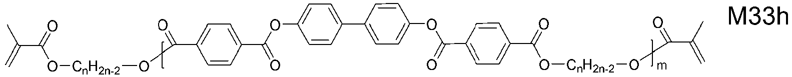 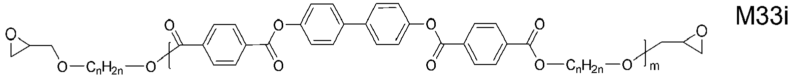 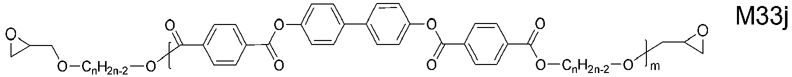 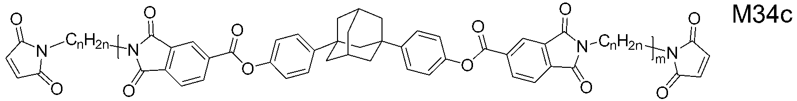 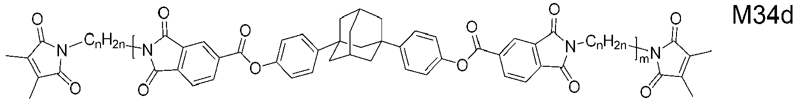 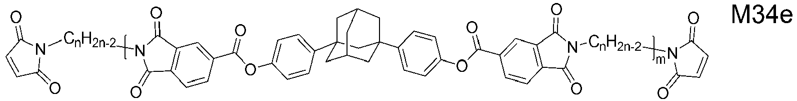 oder 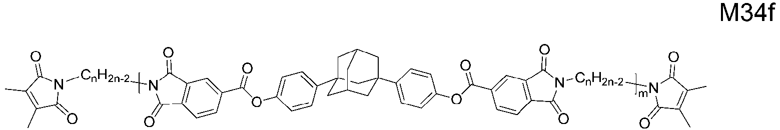 wobei
n eine ganze Zahl von 1 bis 60, vorzugsweise von 1 bis 36 und stärker bevorzugt von 6 bis 12 ist; und
m eine ganze Zahl von 1 bis 60, vorzugsweise 1 bis 50, stärker bevorzugt 2 bis 30 und am stärksten bevorzugt 3 bis 20 ist.

2. Polymerisierbare Verbindung des Anspruchs 1, die aus M34c, M34d, M34e oder M34f ausgewählt ist.

3. Polymerisierbare Verbindung des Anspruchs 1, bei der es sich um 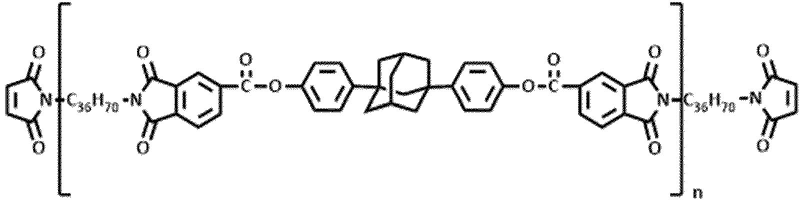 handelt.

4. Verfahren zum Bilden eines Polymers, umfassend die folgenden Schritte:
(i) Bereitstellen eines polymerisierbaren Ausgangsmaterials enthaltend eine oder mehrere polymerisierbare Verbindungen nach irgendeinem der Ansprüche 1 bis 3; und
(ii) Polymerisieren des polymerisierbaren Ausgangsmaterials.

5. Verfahren zum Bilden eines Polymers nach Anspruch 4, wobei das polymerisierbare Ausgangsmaterial ferner eine oder mehrere zusätzliche polymerisierbare Verbindungen enthält, die in der Lage sind, mit der polymerisierbaren Verbindung unter Bildung eines Copolymers zu reagieren.

6. Verfahren zum Bilden eines Polymers nach Anspruch 4 oder 5, wobei das polymerisierbare Ausgangsmaterial in Schritt (ii) durch radikalische oder ionische Polymerisation oder durch Einwirkung von Strahlung polymerisiert wird.

7. Polymer, erhältlich durch das Verfahren zum Bilden eines Polymers nach irgendeinem der Ansprüche 4 bis 6.

8. Elektronische Vorrichtung enthaltend ein Polymer nach Anspruch 7.

9. Elektronische Vorrichtung nach Anspruch 8, wobei das Polymer eine dielektrische Schicht bildet.

10. Herstellungsverfahren zur Herstellung einer verpackten mikroelektronischen Struktur, worin ein Substrat mit einer Beschichtung versehen wird, die ein oder mehrere polymerisierte flüssigkristalline Monomere enthält, wobei das Verfahren die folgenden Schritte umfasst:
(1) Aufbringen einer polymerisierbaren Zusammensetzung enthaltend eine oder mehrere polymerisierbare Verbindungen nach irgendeinem der Ansprüche 1 bis 3 auf eine Oberfläche eines Substrats; und
(2) Härten der polymerisierbaren Zusammensetzung zur Bildung einer dielektrischen Schicht.

11. Herstellungsverfahren zur Herstellung einer verpackten mikroelektronischen Struktur nach Anspruch 10, wobei die polymerisierbare Zusammensetzung ferner eine oder mehrere zusätzliche polymerisierbare Verbindungen enthält, die in der Lage sind, mit der polymerisierbaren Verbindung unter Bildung eines Copolymers zu reagieren.

12. Herstellungsverfahren zur Herstellung einer verpackten mikroelektronischen Struktur nach Anspruch 10 oder 11, wobei die polymerisierbare Zusammensetzung ferner ein oder mehrere anorganische Füllstoffmaterialien enthält.

13. Mikroelektronische Vorrichtung enthaltend eine verpackte mikroelektronische Struktur, die erhältlich ist durch das Herstellungsverfahren nach irgendeinem der Ansprüche 10 bis 12.

## Revendications

1. Composé polymérisable choisi parmi 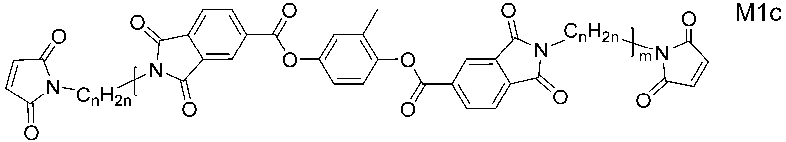 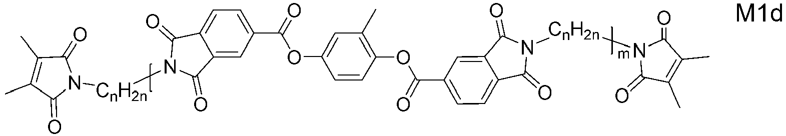 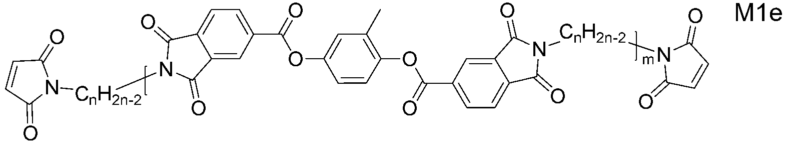 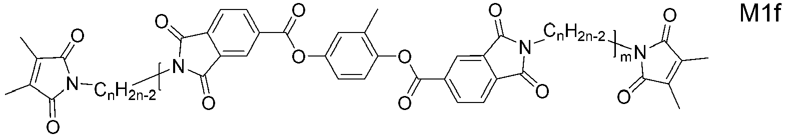 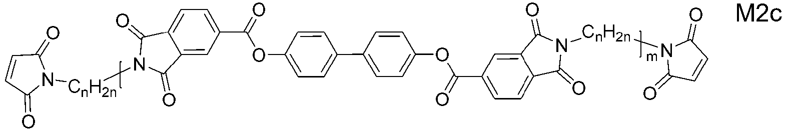 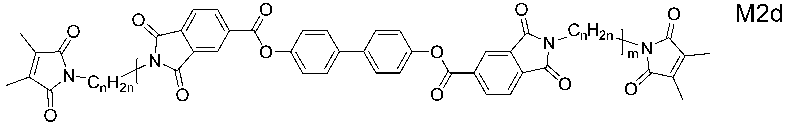 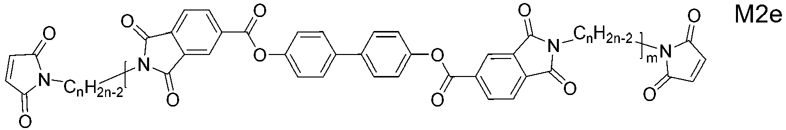 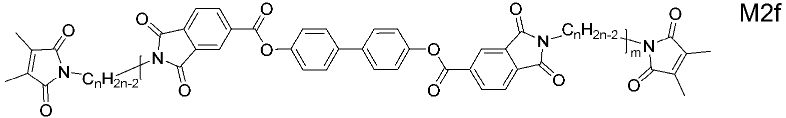 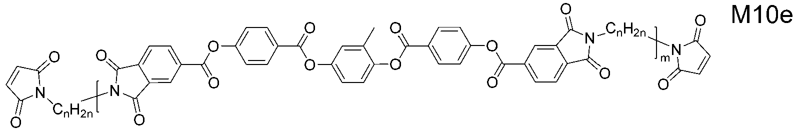 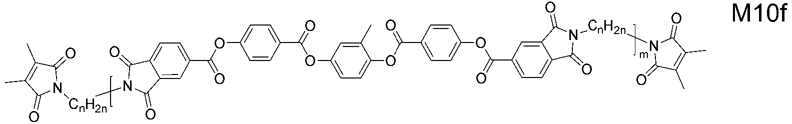 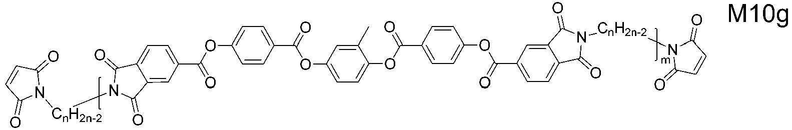 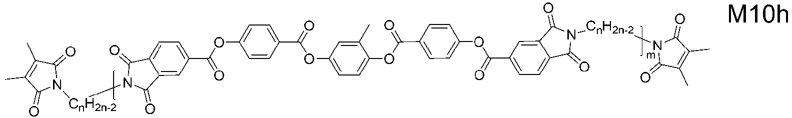 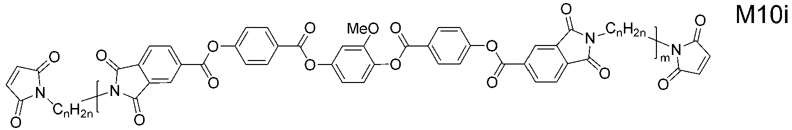 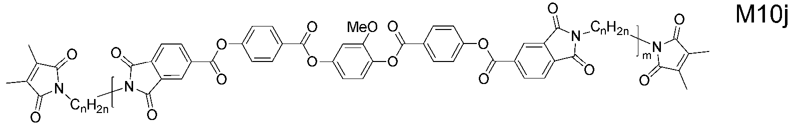 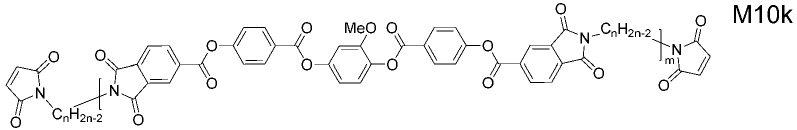 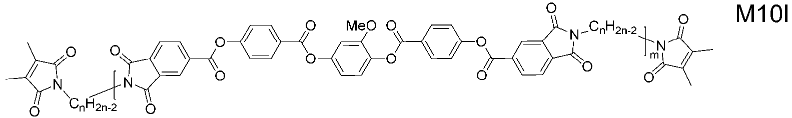 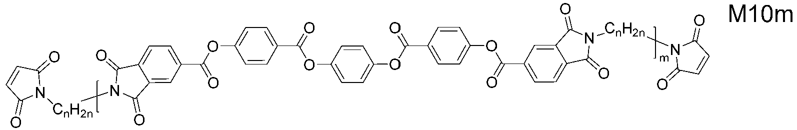 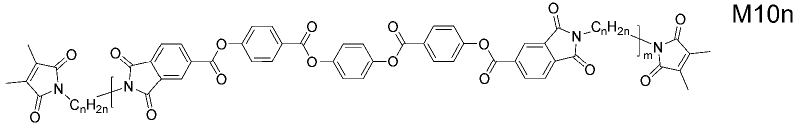 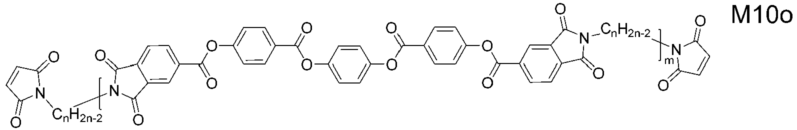 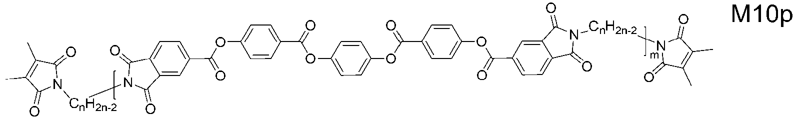 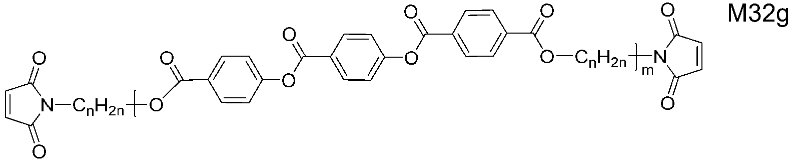 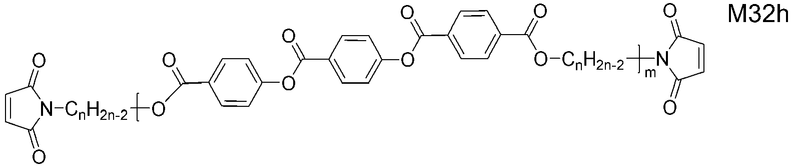 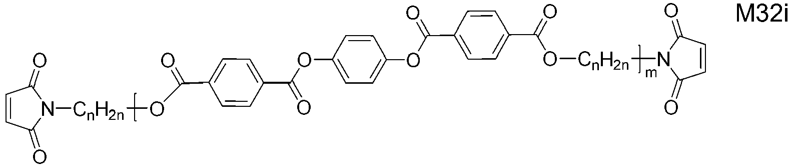 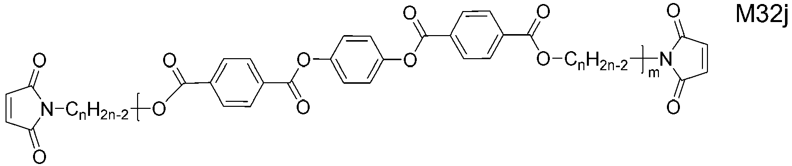 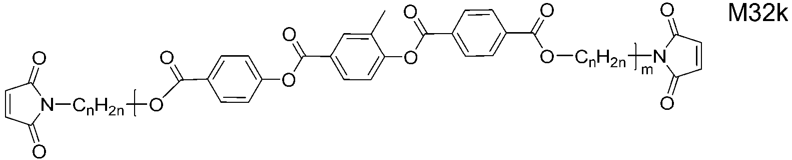 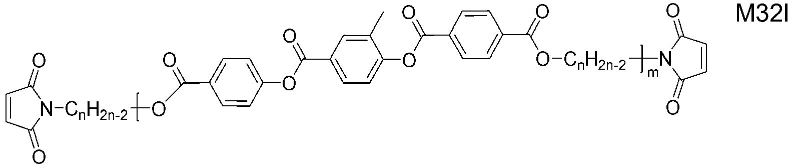 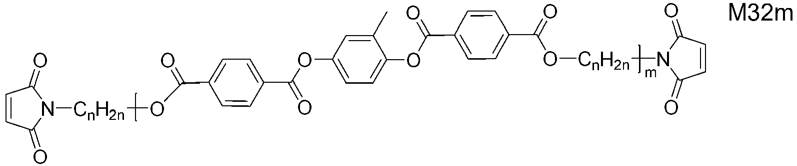 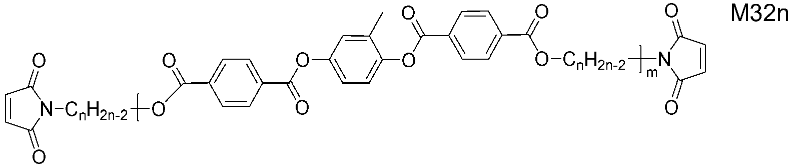 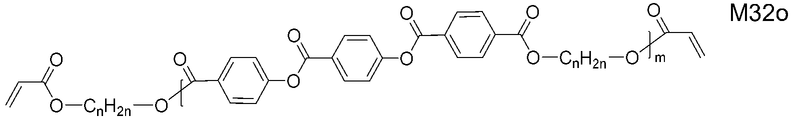 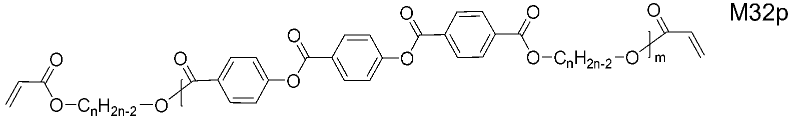 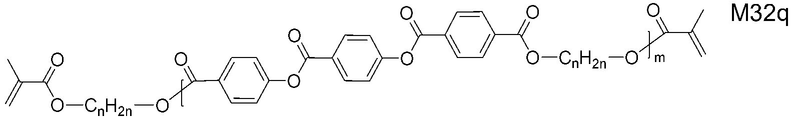 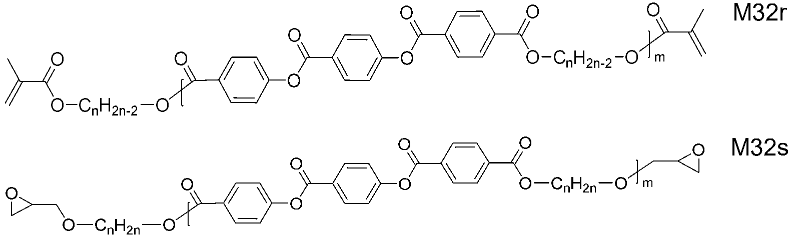 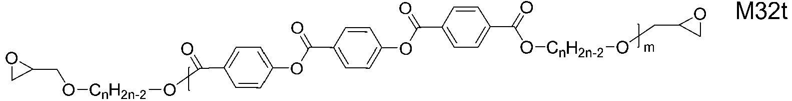 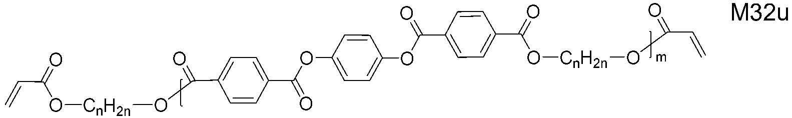 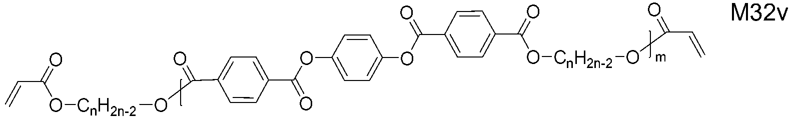 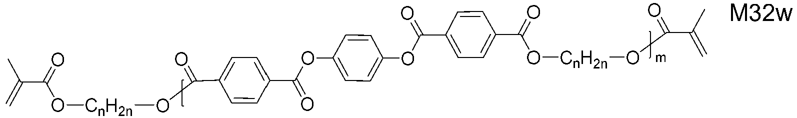 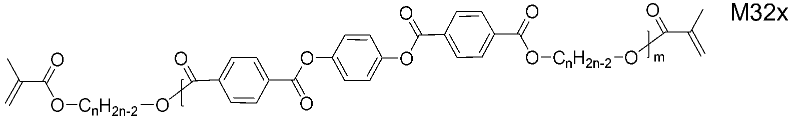 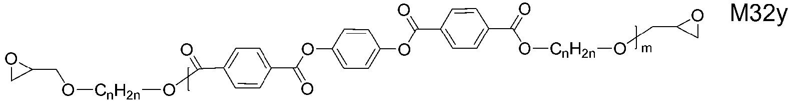 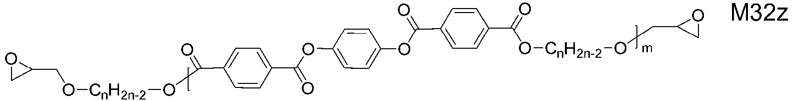 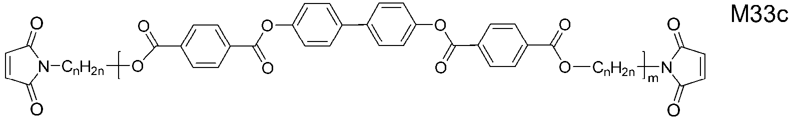 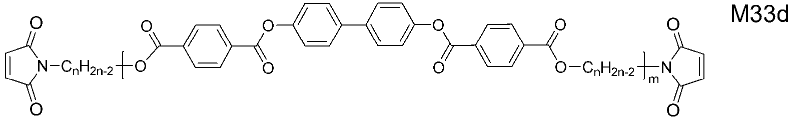 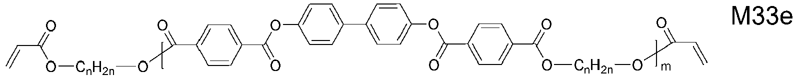 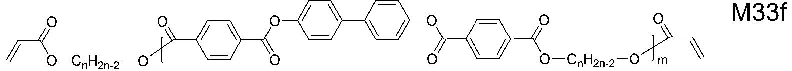 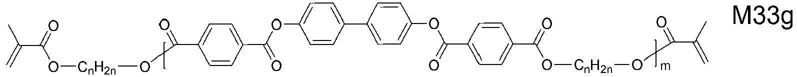 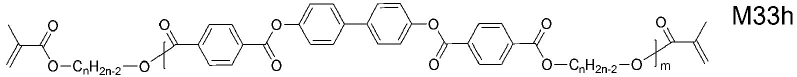 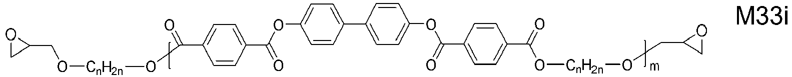 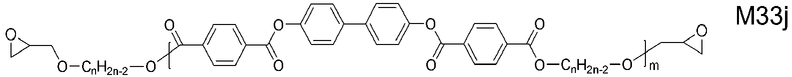 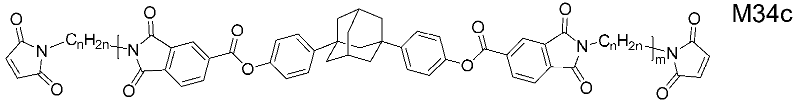 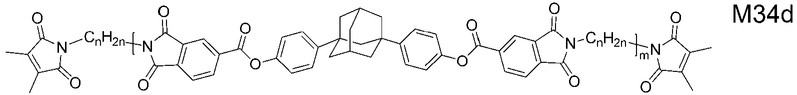 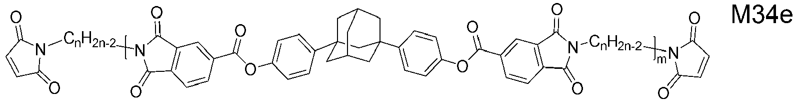 ou 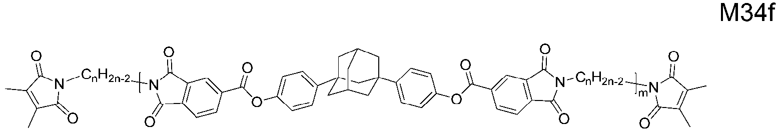 dans lequel
n est un nombre entier allant de 1 à 60, préférablement de 1 à 36, et plus préférablement de 6 à 12 ; et
m est un nombre entier allant de 1 à 60, préférablement de 1 à 50, plus préférablement de 2 à 30, et tout préférablement de 3 à 20.

2. Composé polymérisable selon la revendication 1, étant choisi parmi M34c, M34d, M34e ou M34f.

3. Composé polymérisable selon la revendication 1, étant

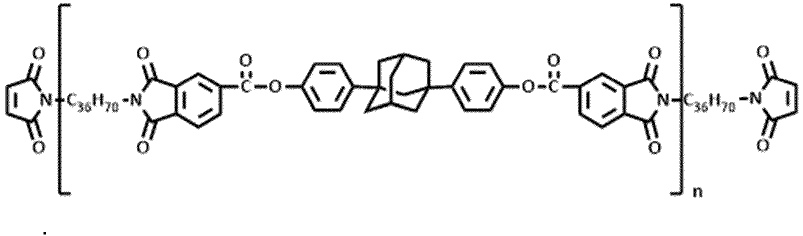

4. Méthode de formation d'un polymère, comprenant les étapes suivantes :
(i) la mise à disposition d'un matériau de départ polymérisable comprenant un ou plusieurs composés polymérisables selon l'une quelconque des revendications 1 à 3 ; et
(ii) la polymérisation dudit matériau de départ polymérisable.

5. Méthode de formation d'un polymère selon la revendication 4, dans laquelle le matériau de départ polymérisable comprend en outre un ou plusieurs composés polymérisables supplémentaires qui sont capables de réagir avec le composé polymérisable afin de former un copolymère.

6. Méthode de formation d'un polymère selon la revendication 4 ou 5, dans laquelle le matériau de départ polymérisable est polymérisé dans l'étape (ii) par polymérisation radicalaire ou ionique ou par exposition à un rayonnement.

7. Polymère, pouvant être obtenu par la méthode de formation d'un polymère selon l'une quelconque des revendications 4 à 6.

8. Dispositif électronique comprenant un polymère selon la revendication 7.

9. Dispositif électronique selon la revendication 8, dans lequel le polymère forme une couche diélectrique.

10. Méthode de fabrication destinée à la préparation d'une structure microélectronique encapsulée, dans laquelle un substrat est muni d'un revêtement comprenant un ou plusieurs monomères cristallins liquides polymérisés, la méthode comprenant les étapes suivantes :
(1) l'application d'une composition polymérisable comprenant un ou plusieurs composés polymérisables selon l'une quelconque des revendications 1 à 3 à une surface d'un substrat ; et
(2) le durcissement de ladite composition polymérisable afin de former une couche diélectrique.

11. Méthode de fabrication destinée à la préparation d'une structure microélectronique encapsulée selon la revendication 10, dans laquelle la composition polymérisable comprend en outre un ou plusieurs composés polymérisables supplémentaires qui sont capables de réagir avec le composé polymérisable afin de former un copolymère.

12. Méthode de fabrication destinée à la préparation d'une structure microélectronique encapsulée selon la revendication 10 ou 11, dans laquelle la composition polymérisable comprend en outre un ou plusieurs matériaux de charge inorganique.

13. Dispositif microélectronique comprenant une structure microélectronique encapsulée pouvant être obtenue par la méthode de fabrication selon l'une quelconque des revendications 10 à 12.
